(19) Europäisches Patentamt
European Patent Office
Office européen des brevets

(11) EP 3 861 988 A1

(12) # EUROPEAN PATENT APPLICATION
published in accordance with Art. 153(4) EPC

(43) Date of publication:
**11.08.2021 Bulletin 2021/32**

(21) Application number: **19868469.8**

(22) Date of filing: **02.10.2019**

(51) Int Cl.:
***A61K 9/14*** *(2006.01)* ***A61K 9/72*** *(2006.01)*
***A61K 47/18*** *(2017.01)* ***A61K 47/26*** *(2006.01)*

(86) International application number:
**PCT/JP2019/038991**

(87) International publication number:
**WO 2020/071448 (09.04.2020 Gazette 2020/15)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.10.2018 JP 2018187498**

(71) Applicant: **Meijo University Educational Foundation**
**Nagoya-shi, Aichi 468-8502 (JP)**

(72) Inventors:
- **OKAMOTO Hirokazu**
**Nagoya-shi, Aichi 468-8502 (JP)**
- **OKUDA Tomoyuki**
**Nagoya-shi, Aichi 468-8502 (JP)**

(74) Representative: **Winter, Brandl - Partnerschaft mbB**
**Alois-Steinecker-Straße 22**
**85354 Freising (DE)**

(54) **POWDER INHALER, METHOD FOR ASSESSING SAME, AND USE OF SAME**

(57) As an inhalation powder medicine excellent in dispersibility, pulmonary delivery, and deposition, there is provided an inhalation powder medicine, containing: an active ingredient in at least a part of the porous hollow spherical particles that are capable of being dispersed and crushed into smaller particles by inspiration and capable of swelling when the porous hollow spherical particles absorb moisture, wherein the smaller particles are also capable of swelling when the smaller particles absorb moisture.

*FIG. 8*

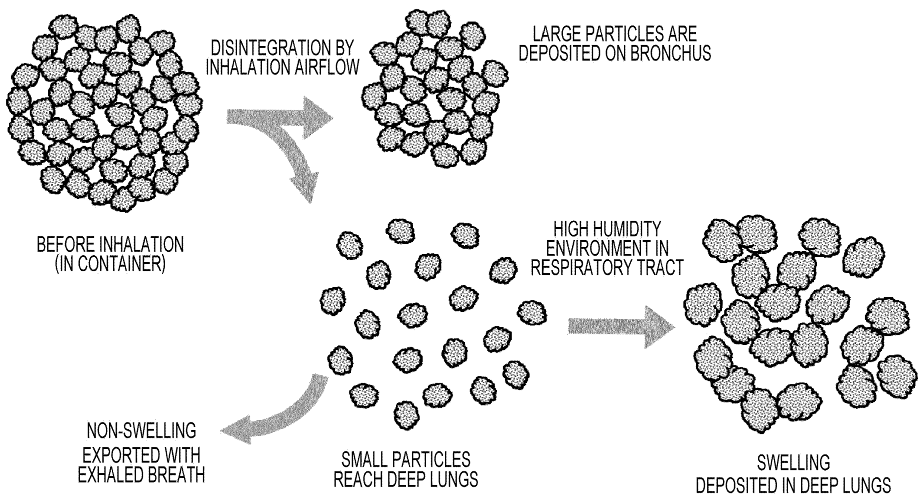




Printed by Jouve, 75001 PARIS (FR)

## Description

Technical Field

The present specification relates to an inhalation powder medicine, a method of evaluation thereof, and use thereof.

(Cross-reference to related applications)

This application is a related application of Japanese Patent Application No. 2018-187498, which is a Japanese patent application filed on October 2, 2018, and claims priority based on this Japanese application. The entire contents thereof are incorporated herein by reference. This application is also a related application of Japanese Patent Application No. 2017-91941, which is a Japanese patent application filed on May 2, 2017. The entire contents thereof are incorporated herein by reference.

Background Art

Lungs are attracting attention as an administration route that is expected to have a systemic effect by not only a local agent but also an agent with low gastrointestinal absorption. Inhalation medicines, which can deliver drugs directly and non-invasively to the lungs, are expected to exhibit their effects quickly, and have an advantage of reduction in systemic side effects because their doses are smaller than those in oral administration (Patent Literature 1). Thus, future use for diseases such as lung cancer and pulmonary hypertension is expected.

Inhalation medicines are classified into three types: Metered-Dose Inhaler (MDI), Inhalation Solution (Inhalation Solution), and Dry Powder Inhaler (DPI). The inhalation method differs depending on each inhaler, and it is important to use it properly to achieve a good therapeutic effect. With DPI, in general, drug powder is disintegrated and dispersed into the air by the patient's inhalation effort and delivered to the respiratory tract treatment area. Thus, from the viewpoint of easy synchronization between powder spraying and inhalation, unnecessity of propellants, and simple inhalation procedure, and the viewpoint of the inhaler being relatively small and excellent in portability, DPI is actively researched and developed in recent years.

Citation List

Patent Literature

Patent Literature 1: Japanese Translation of PCT International Application Publication No. 2007-522246

Summary of Invention

In the case of DPI, a certain level of inhalation ability is required to disperse powder fine particles, and thus there is a problem in that patients capable of using DPI are limited. Further, strong suction is recommended, and thus the agent often adheres to the oral cavity and pharynx. To apply DPI to diseases such as lung cancer and pulmonary hypertension, a formulation design to resolve these problems is necessary.

However, now, no inhalation powder medicine that achieves high dispersibility, pulmonary delivery and deposition is provided.

The present specification provides an inhalation powder medicine excellent in dispersibility, pulmonary delivery, and deposition, a method of evaluation thereof, and use thereof.

Solution to problem

As a result of various formulation studies and evaluations on an inhalation powder medicine that realizes dispersibility, pulmonary delivery, and deposition, the present inventors have found that an inhalation powder medicine that can satisfy these properties can be constructed. The present inventors have also found a method of evaluating such formulation properties. According to the present specification, the following means are provided based on such findings.

[1] An inhalation powder medicine, containing: an active ingredient in at least a part of porous hollow spherical particles that are capable of being dispersed and crushed into smaller particles by inspiration and capable of swelling when the porous hollow spherical particles absorb moisture, wherein the smaller particles are also capable of swelling when the smaller particles absorb moisture.

[2] The inhalation powder medicine according to [1], having OE (%) = recovery amount on and after Throat (mg)/total recovery amount (mg) × 100 of 80% or more in inhalation performance evaluation by Andersen Cascade Impactor (ACI).

[3] The inhalation powder medicine according to [1] or [2], having an FPF5 (%) of 30% or more in inhalation performance evaluation by ACI.

[4] The inhalation powder medicine according to any one of [1] to [3], having a peak of a recovery percentage in any one of filter and Stages 2 to 4 in inhalation performance evaluation by ACI.

[5] The inhalation powder medicine according to any one of [1] to [4], having a first aerodynamic mass median diameter calculated in inhalation performance evaluation by ACI and a second aerodynamic mass median diameter calculated in inhalation performance evaluation by ACI smaller than the first aerodynamic mass median diameter.

[6] The inhalation powder medicine according to [5], having a percentage (mass) of a powder having the second aerodynamic mass median diameter based on a total powder of 40% or more.

[7] The inhalation powder medicine according to any one of [1] to [6], having a mass change rate at 70% RH of 1% or less and a mass change rate at 95% RH of 5% or more when RH is changed from 50% to 95% at 37°C in dynamic vapor sorption measurement.

[8] The inhalation powder medicine according to any one of [1] to [7], wherein the spherical particles contain leucine, mannitol, and trehalose as an excipient.

[9] The inhalation powder medicine according to any one of [1] to [8], wherein the particles have a peak particle size in geometric particle size distribution of 1 μm or more and 100 μm or less.

[10] The inhalation powder medicine according to any one of [1] to [9], containing a nucleic acid as the active ingredient.

[11] The inhalation powder medicine according to [10], wherein the nucleic acid is a naked nucleic acid.

[12] The inhalation powder medicine according to [10] or [11], further containing an anionic component that is an anionic polymer or a salt thereof as an excipient.

[13] The inhalation powder medicine according to [12], wherein the anionic component is hyaluronic acid or a salt thereof.

[14] The powder inhalation medicine according to [13], wherein the hyaluronic acid or a salt thereof has a weight average molecular weight of 30,000 or more and 70,000 or less.

[15] The inhalation powder medicine according to any one of [10] to [14], further containing one or more hydrophobic amino acids as an excipient.

[16] The inhalation powder medicine according to [15], wherein the hydrophobic amino acids are one or more selected from the group consisting of leucine, phenylalanine, and isoleucine.

[17] The inhalation powder medicine according to [10] or [11], containing hyaluronic acid having a weight average molecular weight of 30,000 or more and 70,000 or less or a salt thereof and phenylalanine as an excipient that is a component other than the active ingredient, wherein the inhalation powder medicine has a content of the hyaluronic acid or a salt thereof of 40% by mass or more and 85% by mass or less based on a total mass of these two components.

[18] The inhalation powder medicine according to any one of [10] to [17], for gene expression in a mammal.

[19] The inhalation powder medicine according to any one of [10] to [17], for gene suppression in a mammal.

[20] A method of manufacturing an inhalation powder medicine, including:
a drying step of spray freeze-drying a liquid in which an active ingredient and at least one excipient are dissolved so that porous hollow spherical particles that are capable of being dispersed and crushed into smaller particles by inspiration and capable of swelling when the porous hollow spherical particles absorb moisture, wherein the smaller particles are also capable of swelling when the smaller particles absorb moisture, are obtained.

[21] The method according to claim 20, further including the step of: selecting the at least one excipient and preparing the liquid so that OE (%) = recovery amount on and after Throat (mg)/total recovery amount (mg) × 100 is 80% or more in inhalation performance evaluation by Andersen Cascade Impactor (ACI) of the spherical particles.

[22] The method according to [21], wherein the at least one excipient is selected and the liquid is prepared so that an FPF5 (%) is 30% or more in inhalation performance evaluation by ACI of the spherical particles.

[23] The method according to any one of [20] to [22], wherein the at least one excipient is selected and the liquid is prepared so that spherical particles having a peak of a recovery percentage in any one of Filter and Stages 2 to 4 in inhalation performance evaluation by ACI of the spherical particles are obtained.

[24] The method according to any one of [20] to [23], wherein the drying step is a step of drying a liquid containing an active ingredient by a spray freeze-drying method using leucine, mannitol, and trehalose as the at least one excipient.

[25] A method of evaluating an inhalation powder medicine, including the step of:
obtaining a first aerodynamic mass median diameter calculated in inhalation performance evaluation by ACI and a second aerodynamic mass median diameter calculated in inhalation performance evaluation by ACI smaller than the first aerodynamic mass median diameter.

[26] The method according to [25], wherein a percentage (mass) of a powder having the second aerodynamic mass median diameter based on a total powder is obtained.

[27] A method of evaluating an inhalation powder medicine, including the step of:
measuring at least a mass change rate at 70% RH and a mass change rate at 95% RH when RH is changed from 50% to 95% at 37°C in dynamic vapor sorption measurement.

Brief Description of Drawings

Fig. 1 is a diagram showing an outline of inhalation performance evaluation by Andersen Cascade Impactor (ACI).
Fig. 2 is a diagram showing the results of electron microscope (SEM) observation of the inhalation powder medicine prepared in Example 1.
Fig. 3 is a diagram showing a particle size distribution and a $D_{50}$ diameter of the inhalation powder medicine prepared in Example 1.
Fig. 4 is a diagram showing the recovery percentage of the inhalation powder medicine prepared in Example 1 at each site by the ACI method.
Fig. 5 is a diagram showing an index of inhalation performances of the inhalation powder medicine prepared in Example 1.
Fig. 6 is a diagram showing the aerodynamic mass median diameter (MMAD), geometric standard deviation (GSD), and disintegration ratio (R) of the inhalation powder medicine prepared in Example 1.
Fig. 7 is a diagram showing the measurement results of anti-hygroscopic property and hygroscopic property by a dynamic vapor sorption measuring instrument.
Fig. 8 is a diagram showing an outline of an effect aspect of the inhalation powder medicine.
Fig. 9A is a diagram showing the relationship between the storage conditions and the inhalation performance evaluation of the inhalation powder medicine containing hyaluronic acid/leucine as excipients.
Fig. 9B is a diagram showing the relationship between the storage condition and the inhalation performance evaluation of the inhalation powder medicine containing only hyaluronic acid as an excipient.
Fig. 10 is a diagram showing the relationship between the storage condition and the efficacy (gene expression when suction administration was performed to a mouse) of the inhalation powder medicine containing hyaluronic acid/leucine and the inhalation powder medicine containing hyaluronic acid as an excipient.
Fig. 11 is a diagram showing the influence of the molecular weight of hyaluronic acid on the gene expression (in vitro) of the inhalation powder medicine containing hyaluronic acid/phenylalanine as excipients.
Fig. 12 is a diagram showing the evaluation results of gene expression (in vitro) of the inhalation powder medicine containing hyaluronic acid (sodium salt) having a weight average molecular weight of 50,000 and phenylalanine in various ratios as an excipient.

Fig. 13 is a diagram showing the evaluation results of deposition percentage, inhalation performance, and MMAD when hyaluronic acid (sodium salt) having a weight average molecular weight of 50,000 and phenylalanine are used as an excipient.

Fig. 14 is a diagram of estimation of the efficacy when hyaluronic acid (sodium salt) having a weight average molecular weight of 50,000 and phenylalanine are used as an excipient.

Description of Embodiments

The disclosure of the present specification relates to an inhalation powder medicine, a method of evaluation thereof, and use thereof. According to the inhalation powder medicine disclosed in the present specification, the inhalation powder medicine is dispersed and crushed into smaller particles by inspiration, and thus is excellent in dispersibility and reachability to the lungs. Further, the inhalation powder medicine and the smaller particles absorb moisture and swell in a high humidity environment, and exhibit adhesiveness and aggregability in the lungs.

According to the method of evaluating an inhalation powder medicine disclosed in the present specification, an inhalation powder medicine excellent in dispersibility, reachability to the lungs, and adhesiveness can be evaluated, and the properties thereof can be controlled.

According to the inhalation powder medicine disclosed in the present specification, a pharmaceutical composition useful for preventing and treating diseases and disorders in the lungs can be provided by targeting the lungs and organs around the lungs.

Hereinafter, typical and non-limiting specific examples of the present disclosure will be described in detail with reference to the drawings as appropriate. This detailed description is intended to provide those skilled in the art with details for implementing the preferable examples of the present disclosure and is not intended to limit the scope of the present disclosure. The additional features and inventions disclosed below can be used separately or together with other features and inventions to provide a further improved "inhalation powder medicine and a method of evaluation thereof" and the like.

The combination of features and processes disclosed in the following detailed description is not essential in performing the present disclosure in the broadest sense, and is particularly described to explain typical specific examples of the present disclosure. The various features of the typical specific examples above and below, as well as the various features of those described in the independent and dependent claims must not be combined according to the specific examples described herein or in the order listed to provide additional and useful embodiments of the present disclosure.

All features described in the present specification and/or Claims are intended to be disclosed separately and independently of each other as a limitation to the disclosure at the time of filing and the specific matters claimed, apart from the structure of the features described in Examples and/or Claims. In addition, all numerical ranges and descriptions relating to groups are intended to disclose their intermediate structure as a limitation to the disclosure at the time of filing and the specific matters claimed.

(Inhalation powder medicine)

The inhalation powder medicine (hereinafter, also simply referred to as the present powder medicine) disclosed in the present specification can contain an active ingredient in at least a part of porous hollow spherical particles that are capable of being dispersed and crushed into smaller particles by inspiration and capable of swelling when the porous hollow spherical particles absorb moisture, wherein the smaller particles are also capable of swelling when the smaller particles absorb moisture. Hereinafter, various properties of the present powder medicine and the method of evaluation thereof will be described, and then the method of manufacturing the present powder medicine will be described.

(Particle size of present powder medicine)

The particle size of the present powder medicine can be measured by a dry laser diffractometry. The 50% particle size ($D_{50}$) can be calculated from the cumulative particle size distribution curve. For example, it can be measured using a laser micron sizer (LMS-2000e) or an instrument equivalent to that. Though the 50% particle size of the present powder medicine is not particularly limited, it can be, for example, 1 μm or more and 100 μm or less, 2 μm or more and 50 μm or less, and 5 μm or more and 20 μm or less, considering the scattering property and dispersibility. It can be also, for example, 5 μm or more and 20 μm or less, and for example, 5 μm or more and 15 μm or less.

(Particle shape of the present powder medicine)

The particle shape of the present powder medicine can be observed with a scanning electron microscope. For the observation, for example, the powder is sprayed on a sample mount using a powder fine particle addition device used for the dispersion addition of the present powder medicine, and then platinum coating for SEM observation is applied as necessary and observed. As the powder fine particle addition device and the spraying method, for example, those used in the Examples described later can be employed.

Though the particle shape of the present powder medicine is not particularly limited, it is preferably spherical considering the scattering property, dispersibility and the like. It is preferably porous considering the scattering property, swelling property and the like. Further, it preferably has a hollow structure. Typically, the present powder medicine can be, for example, porous spherical particles having a large number of pores (hollow portions) formed by a partition wall composed of constituent components such as an active ingredient and an excipient of the present powder medicine formed by sublimation of water.

(Inhalation performances)

The present powder medicine is delivered to the respiratory tract by inspiration (gas flow during suction from the oral cavity to the bronchus). The properties at that time (inhalation performances), i.e., the dispersibility, delivering property, and disintegration property of the present powder medicine can be evaluated by evaluation by Andersen Cascade Impactor (ACI) method. Dispersibility, delivering property, and disintegration property are each independent property, but are interrelated.

(Evaluation method by Andersen Cascade Impactor (ACI) method)

In the present specification, in the ACI method, a measuring instrument described in 5.2 Andersen Cascade Impactor Method (Apparatus 2), 6.15 Aerodynamic Particle Size Measurement for Inhalations in Supplement I to the Japanese Pharmacopoeia, 17th Edition, General Tests, Processes and Apparatus is used. A pre-separator can be used as appropriate. Examples of the measuring instrument include a low volume air sampler, Andersen type, AN-200 type, an instrument manufactured by SIBATA SCIENTIFIC TECHNOLOGY LTD.

Fig. 1 shows an outline of the measuring instrument and an example of the measuring method. As shown in Fig. 1, the measuring instrument is equipped with Device that is an introduction part, Throat, eight Stages from Stage 0 to Stage 7, and Filter at the bottom. Each Stage has a filter structure and is configured to classify and capture particles so that the lower the Stage, the smaller the aerodynamic particle size. For the particle size ($\mu$m) of the particles classified in each Stage, for example, the size shown in Fig. 1 can be employed as the cutoff size when the suction amount is 28.3 L/min. In Fig. 1, the respiratory organs corresponding to each Stage are also described.

The evaluation of the present powder medicine by the ACI method can be performed according to the measurement procedure of 5.2.2 Procedure for dry powder inhalers, 5.2 Andersen Cascade Impactor Method (Apparatus 2) of the above-mentioned General Tests, Processes and Apparatus. That is, it can be performed with a flow rate of 28.3 L/min and an air volume of 4 L. The inhalation resistance is also appropriately selected by the inhalation device.

After the measurement, the mass of the present powder medicine (particles or active ingredient) on the capsule used for introduction, Device, Throat, each Stage and Filter is measured. The amount of the present powder medicine can be measured not only by quantitatively detecting the active ingredient, but also by, for example, adding an appropriate label to the particles for evaluation purposes and measuring the label. Quantification of the active ingredient can be performed by those skilled in the art as needed, and the use and detection of such a label are well known to those skilled in the art.

(Dispersibility and delivery properties)

The dispersibility and delivering property of the present powder medicine can be evaluated using the numerical values and the like obtained by the following formula as an index. In the present specification, Stage 3 and Stages after Stage 3 are defined as an intrapulmonary delivery region effective for inhalation medicine application, and Stage 5 and Stages after Stage 5 are defined as a deep lung delivery region where systemic action can be expected.

OE (Output Efficiency: %), which is the release percentage from the Device, is calculated by the following formula (1).

FPF $_{stage3}$ (Fine Particle Fraction: %) which indicate the percentage of the present powder medicine that reached Stage 3 and after Stage 3, and FPF $_{stage5}$ which indicate the percentage of the present powder medicine that reached Stage 5 and after Stage 5 in the powder released from the Device can be calculated from Formulas (2) and (3), respectively. OE $\times$ FPF $_{Stage5}$ (%), which indicates the percentage of the powder that reached Stage 5 and Stages after Stage 5 in the total recovery amount, can be calculated from the formula (4).

$$OE\ (\%) = \text{Recovery amount T } (\mu g)/\text{Total recovery amount } (\mu g) \times 100 \quad (1)$$

(the recovery amount T is a recovery amount on and after Throat.)

$$FPF_{Stage3}\ (\%)\ (FPF3) = \text{Recovery amount on and after Stage 3 } (\mu g)/\text{Recovery amount T } (\mu g) \times 100 \quad (2)$$

$$FPF_{Stage5}\ (\%)\ (FPF5) = \text{Recovery amount on and after Stage 5 } (\mu g)/\text{Recovery amount T } (\mu g) \times 100 \quad (3)$$

$$OE \times FPF_{Stage5}\ (\%) = \text{Recovery amount on and after Stage 5 } (\mu g)/\text{Total recovery amount } (\mu g) \times 100 \quad (4)$$

OE is an index of dispersibility, FPF3 is an index of intrapulmonary delivering property, and FPF5 is an index of deep lung delivering property.

Because each Stage can correspond to each respiratory tract, the percentage of the recovery amount (recovery percentage) of the present powder medicine in each site or Stage of the instrument based on the total recovery amount from the measuring instrument can be used as an index of the delivery percentage to the corresponding respiratory tract site.

The present powder medicine can have, for example, an OE of 80% or more in the inhalation performance evaluation by the ACI method. This is because an OE of 80% or more can mean a good release percentage. The OE is also, for example, 85% or more, for example, 90% or more, and, for example, 95% or more.

In the inhalation performance evaluation by the ACI method, the present powder medicine can have, for example, an FPF3 of 20% or more, for example, an FPF3 of 30% or more, and for example, an FPF3 of 40% or more. This is because an FPF3 of 40% or more can mean an extremely good delivery percentage to the lungs. The FPF3 is, for example, 50% or more, for example, 60% or more, for example, 70% or more, for example, 80% or more, and, for example, 90% or more. Depending on the active ingredient and use of the inhalation powder medicine, an FPF3 of 20% or more may be sufficient.

In the inhalation performance evaluation by the ACI method, the present powder medicine can have, for example, an FPF5 of 10% or more, for example, an FPF5 of 15% or more, for example, an FPF5 of 20% or more, for example, an FPF5 of 25% or more, and for example, an FPF5 of 30% or more. This is because an FPF5 of 30% or more can mean an extremely good delivery percentage to the deep lungs. The FPF5 is, for example, 40% or more, for example, 50% or more, for example, 55% or more, for example, 60% or more, and, for example, 65% or more. Depending on the active ingredient and use of the inhalation powder medicine, an FPF5 of 10% or more may be sufficient.

The present powder medicine can have a peak of a recovery percentage at any of Stages 2 to 4 and the Filter in the inhalation performance evaluation by the ACI method. This is because such a recovery percentage property can mean that the present powder medicine is excellent in crushability or disintegration property, also excellent in the swelling property by moisture absorption, and excellent in delivering property to deep lungs. Typically, the Stage 3 and the Filter can have a peak of the recovery percentage. Typically, the peak of a recovery percentage on the Filter is greater than the other peak, and is greater by, for example, 30% or more, and, for example, 40% or more.

(Disintegration property)

According to the present inventors, the disintegration property of the present powder medicine can be evaluated by the ACI method. The disintegration property of the present powder medicine can be determined from the disintegration ratio and the aerodynamic mass median diameter of the present powder medicine.

Such property evaluation is useful in the present powder medicine, presumably because in the present powder medicine, some of the particles are disintegrated by suction, particles formed by partial disintegration of the particles having the original size are produced, and the particles having the original size and the particles that are disintegrated are present in a mixed manner. The outline of this reasoning is shown in Fig. 8. As shown in Fig. 8, particles having an original size are partially disintegrated by the inhalation airflow, particles that remain relatively large will adhere to, for example, near bronchi of the lungs, and particles that have disintegrated and have a small particle size will further flow deep into the lungs.

The particle size distribution of the powder is empirically based on a lognormal distribution, a straight line is obtained when the cumulative value of the recovery percentage for each Stage is plotted against the logarithmic value of the cutoff size, the 50% particle size is taken as MMAD, and (84.3% particle size)/(50% particle size) is taken as the geometric standard deviation (GSD). It can be said that the present powder medicine has a property of the lognormal plot being not a straight line but a curved line.

In the present specification, the powder having a large particle size (having aerodynamic mass median diameter $MMAD_c$ and the geometric standard deviation $GSD_c$ thereof) and the powder having a small particle size (having aerodynamic mass median diameter $MMAD_f$ and the geometric standard deviation $GSD_f$ thereof) are thought to be present in a ratio of (1 - R): R (disintegration ratio) to determine the disintegration ratio R, the aerodynamic mass median diameter $MMAD_{f, c}$, and the geometric standard deviation $GSD_{f, c}$ thereof.

(Disintegration ratio and aerodynamic mass median diameter)

From the cumulative value of the recovery percentage (% in the amount released) at 8 measurement points (Stage 1 to Filter) obtained by the ACI method, the values A to D below are determined for each Stage, and R, $MMAD_c$, $GSD_c$, $MMAD_f$, and $GSD_f$ are determined so that E will take the minimal value (see Table 1). For the calculation of each numerical value A to D which minimizes E, for example, the following functions and solver function of spreadsheet software Excel (Microsoft Corporation) can be used. As the cutoff value, the numerical value shown in Fig. 1 was used.

[Table 1]

| Name | | Abbreviation |
|---|---|---|
| Disintegration ratio | | R |
| Large powder | Aerodynamic mass median diameter | $MMAD_c$ |
| | Geometric standard deviation | $GSD_c$ |
| Small powder | Aerodynamic mass median diameter | $MMAD_f$ |
| | Geometric standard deviation | $GSD_f$ |

A = Random variable obtained by converting the cumulative value (%) of the recovery percentage for each Stage by the NORM.S.INV function.

B = Cumulative value of the recovery percentage of particles having a large particle size ($MMAD_c$ and $GSD_c$) calculated by NORM.DIST function

C = Cumulative value of the recovery percentage of powder of particles having a small particle size ($MMAD_f$ and $GSD_f$) calculated by NORM.DIST function

Random variable obtained by converting the value of D = B × (1-R) + C × R by the NORM.S.INV function

E = Total value of $(D-A)^2$ determined for each Stage

Based on the above, it can be said that the present powder medicine can have the aerodynamic mass median diameter $MMAD_c$ (first aerodynamic mass median diameter) and the second aerodynamic mass median diameter $MMAD_f$ smaller than the first aerodynamic mass median diameter (second aerodynamic mass median diameter) calculated under the above conditions in the inhalation performance evaluation by the ACI method.

The present powder medicine can have a ratio (%) (disintegration ratio) of the powder having the second aerodynamic mass median diameter based on the total mass of the total powders (powder having the first aerodynamic mass median diameter and powder having the second aerodynamic mass median diameter) of, for example, 40% or more. This is because a ratio of 40% or more can mean a high delivery percentage to the deep lungs. The disintegration ratio is also, for example, 44% or more, for example, 50% or more, for example, 55% or more, and, for example, 60% or more.

(Hygroscopic property/swelling property)

The present powder medicine can also have a mass change rate of, for example, 2% or less at 70% RH when RH is changed from 50% to 95% at 37°C in dynamic vapor sorption measurement. This is because a mass change rate of 2% or less can mean a sufficient anti-hygroscopic property before inhalation. For example, the mass change rate is 1.5% or less, and for example, 1% or less.

For example, the mass change rate at 95% RH can be 8% or more. This is because a mass change rate of 8% or more means high hygroscopicity. Such mass change rate is also, for example, 9% or more, for example, 10% or more, and for example, 11% or more.

The dynamic vapor sorption measurement is a method in which a dynamic vapor sorption measuring instrument (DVS: Dynamic Vapor Sorption; DVS advantage, Surface Measurement Systems) is used, which monitors the mass change rate of the sample on the balance due to the adsorption and desorption of moisture in the preset temperature and humidity environment on a second scale. Anti-hygroscopic property and hygroscopic growth can be evaluated from inhalation to the respiratory tract and deep lungs with high humidity. In the present specification, evaluation can be performed in the environment before inhalation of "temperature of 37°C, relative humidity (RH) of 50% (absolute humidity: 6.903 g/$m^3$)", and the environment in the lungs after inhalation of "temperature of 37°C, RH of 95% (absolute humidity: 41.62 g/$m^3$)".

Regarding hygroscopic property and swelling property, after evaluation by the ACI method under normal conditions (that is, dry conditions, about 40% RH or less), particles on each Stage are collected as dry particles, then humidified air (for example, 90% RH or more) is sucked in, then particles on each Stage are collected as humidified particles, and particle shapes of these particles are each observed with SEM and the like to evaluate the hygroscopic property and swelling property.

Alternatively, in the ACI method, a box for adjusting the dry/humidified conditions is placed between Device and Throat, and the ACI method is performed under the same conditions except that the box is set to the dry condition and the humidifying condition. By evaluating the resulting recovery percentage for each Stage, the hygroscopic property and expansivity of particles can be evaluated.

Further, after spraying the present powder medicine on the sample mount of a scanning electron microscope, the powder is exposed to a water bath at 37°C for a short time (for example, within several seconds to several tens of seconds) and observed by SEM. Hygroscopic property and expansivity can be evaluated from the change in particle shape before and after exposure to water vapor.

As shown in Fig. 8, due to appropriate hygroscopicity and swelling property, disintegration property can be ensured, and the powder can be deposited in the deep lungs by moisture absorption and swelling in the respiratory tract or the like after disintegration at the time of inhalation.

The present powder medicine can have advantageous properties in one or more indexes of the properties described above. The present powder medicine is excellent in dispersibility, pulmonary delivery, and deposition, and is useful as an inhalation powder medicine.

(Composition of the present powder medicine and method of production thereof)

The present powder medicine is generally intended for pharmaceutical use and can contain a pharmaceutically acceptable excipient. The excipient is not particularly limited. The powder can contain, for example, one or more selected from leucine, mannitol, and trehalose. Preferably, three excipients are used. These three excipients can each contribute to the above-mentioned advantageous properties of the present powder medicine. Leucine, mannitol, and trehalose are not particularly limited, and all of them can be natural types, that is, L-leucine, D- (-)-mannitol, and D- (+)-trehalose.

For example, leucine can contribute to anti-hygroscopic property and high dispersibility. The present inventors have already reported this (Chem. Pharm. Bull. 64, 239-245 (2016)). Mannitol can contribute to the disintegration property. Trehalose can contribute to the hygroscopicity and swelling property. Mannitol and trehalose can contribute to hygroscopicity and swelling property under high humidity. Thus, by appropriately combining these three, a preparation having excellent hygroscopic property during storage, good dispersibility and disintegration property during suction, and an excellent hygroscopic property and swelling property in a high humidity environment corresponding to the lungs can be provided.

The contents of leucine, mannitol, and trehalose in the present powder medicine are not particularly limited, and for example, the mass ratio of mannitol: trehalose: leucine can be 0 or more and 10 or less: 0 or more and 5 or less: 85 or more and 100 or less. For example, it can be preferably more than 0 and 10 or less: more than 0 and 5 or less: 85 or more and 100 or less, and preferably 5 or more and 10 or less: 1 or more and 5 or less: 85 or more and 94 or less. In addition to the above-mentioned excipients, known excipients can be appropriately used for the present powder medicine.

(Anionic component)

The present powder medicine can also have an anionic component that is an anionic polymer or a salt thereof as an excipient. Though the mechanism is not always clear, presumably, the anionic component can increase the efficiency of introducing the nucleic acid into a cell when the anionic component coexists with the nucleic acid which is a solid substance as an active ingredient. It is presumed that the anionic component contributes to the maintenance of the biological activity of the nucleic acid when the anionic component coexists with the nucleic acid in the present powder medicine during drying by spray freeze-drying or the like. Such an anionic component and the usefulness are disclosed in Japanese Patent Laid-open Publication No. 2018-11588 by the present inventors.

The anionic polymer is not particularly limited, and examples thereof include a negatively charged, naturally derived or synthetic polymer having a molecular weight of about 5 to 4 million and containing an anionic group in the molecule. The anionic group is not particularly limited, a polymer having multiple, preferably 5 or more anionic groups in one molecule can be used, and examples of such a functional group include a carboxyl group, a $-OSO_3H$ group, a $-SO_3H$ group, and a phosphate group. Such an anionic polymer also includes a zwitterionic polymer.

More specific examples of the anionic polymer include a polysaccharide having an anionic group or a derivative thereof; a polypeptide containing an amino acid residue having an anionic group in the side chain; a PEG derivative having a carboxyl side chain; and a synthetic polymer having an anionic group.

Examples of the polysaccharide having an anionic group or a derivative thereof include a glucosaminoglycan. The molecular weight of such a glucosaminoglycan is preferably 10 to 4 million, and more preferably 40 to 3 million. Specific examples of the glucosaminoglycan include hyaluronic acid, chondroitin, chondroitin sulfate, carboxymethyl cellulose, keratan sulfate, heparin, and dermatan sulfate. Among them, hyaluronic acid is presumed to have an excellent contribution to nucleic acid introduction and protection. Derivatives of various glucosaminoglycans such as hyaluronic acid include those obtained by introduction of polyethylene glycol, peptides, sugars, proteins, iodide, antibodies or a part thereof, and a zwitterionic derivative having a positively charged moiety by introduction of spermine, spermidine and the like.

Hyaluronic acid or a salt thereof having a wide range of molecular weight can be used regardless of the origin thereof. For example, the average molecular weight (typically, weight average molecular weight) of hyaluronic acid is suitably 5,000 or less (less than 5,000), and the average molecular weight can be 10,000 or more, or 20,000 or more, and further, 30,000 or more. The average molecular weight can be 40,000 or more. The upper limit is not particularly limited, and for example, the average molecular weight can be 200,000 or less, or 150,000 or less. For example, the hyaluronic acid having an average molecular weight of 50,000 or more and 110,000 or less can be also suitably used. As such hyaluronic acid or a salt thereof (for example, sodium salt), for example, FCH-SU (molecular weight: 50,000 to 110,000) and microhyaluronic acid FCH (molecular weight: 5000 or less (or less than 5000) (both manufactured by Kikkoman Biochemifa Company)) and the like can be used as appropriate.

The weight average molecular weight of hyaluronic acid is suitably 15,000 or more and 40,000 or less. By using such hyaluronic acid or a salt thereof, the efficiency of introducing naked nucleic acids such as siRNA may be increased.

The weight average molecular weight of hyaluronic acid can be 30,000 or more and 70,000 or less, and can be 40,000 or more and 60,000 or less. By using such hyaluronic acid or a salt thereof, the biological activity of the nucleic acid as an active ingredient (for example, the expression level thereof when the nucleic acid is an expression cassette) can be sufficiently highly exhibited, and appropriate inhalation performances can be exhibited.

The average molecular weight of hyaluronic acid can be determined, for example, by a method of combining size exclusion chromatography and a multi-angle light scattering detector (SEC/MALS, for example, "National Institute of Pharmaceutical and Food Sanitation Report", 2003, Vol. 121, p.30-33) and a combination of the Morgan-Elson method and the Carbazol sulfuric acid method (see Patent Literature: Japanese Patent Laid-open Publication No. 2009-155486). SEC/MALS is preferably used.

Examples of the polypeptide containing an amino acid residue having an anionic group in the side chain include a peptide having a molecular weight of 5 to 1 million. Specific examples of such a polypeptide include polyglutamic acid and polyaspartic acid.

Examples of the PEG derivative having a carboxyl side chain include a PEG derivative having multiple, preferably 5 or more carboxyl side chains per PEG molecule, and having a molecular weight of 500 or more, preferably having a molecular weight of 2,000 or more, and more preferably having a molecular weight of 4,000 to 40,000.

The synthetic polymer having an anionic group is a polymer or a copolymer having multiple, preferably 5 or more anionic groups per molecule, and preferably having a molecular weight of 5 to 4 million. Specific examples of such a polymer or copolymer include a polymer or copolymer of acrylic acid or methacrylic acid having a molecular weight of 10 to 3 million, and a sulfate ester of polyvinyl alcohol, and succinimidylated poly-L-lysine.

Examples of the anionic polymer salt include salts of alkali metals such as potassium and sodium, salts of alkaline earth metals such as calcium and magnesium, and ammonium salts. The salt is appropriately selected according to the anionic polymer used.

In the present powder medicine, one or more anionic components of various aspects (that is, type of polymer, molecular weight, type of salt and the like) can be appropriately combined and used as an excipient. As will be described later, as the anionic component used in the present powder medicine, those commercially obtained as appropriate, those artificially synthesized as needed, and appropriate combinations thereof can be used as long as they can improve, for example, stabilization of a nucleic acid as a solid substance, introduction into a cell, and the expression of functions peculiar to the nucleic acid such as gene expression or suppression in a cell.

The compounding ratio of the nucleic acid and the anionic component in the present powder medicine is not particularly limited, depends on the type of the anionic component and the presence or absence of an excipient that acts as a dispersion aid described later, and can be, for example, 5 parts by mass or more and 100 parts by mass or less of the anionic component to 1 part by mass of the nucleic acid. The compounding ratio is more preferably 5 parts by mass or more and 50 parts by mass or less of the anionic component. For example, the compounding ratio is 25 parts by mass or more and 45 parts by mass or less of the anionic component, for example, 30 parts by mass or more and 43 parts by mass or less of the anionic component, for example, 25 parts by mass or more and 40 parts by mass or less of the anionic component, and for example, 30 parts by mass or more and 43 parts by mass or less of the anionic component.

(Hydrophobic amino acid)

The present powder medicine can further contain one or more hydrophobic amino acids as an excipient. Presumably, when such amino acids are contained, for example, the dispersibility when the present powder medicine is supplied to a cell and the inhalation performances at the time of inhalation administration can be improved. Such hydrophobic amino acids and their usefulness are disclosed in Japanese Patent Application Laid-Open No. 2018-11588 by the present inventors.

Examples of the hydrophobic amino acid include leucine, isoleucine, valine, glycine, proline, alanine, tryptophan, phenylalanine, and methionine. Of these, leucine and phenylalanine are preferably used. Presumably, the dispersibility and the like of the active ingredient such as a nucleic acid and anionic components existing as a solid phase can be improved by suitable hydrophobicity. For example, phenylalanine presumably contributes to the suitable cell introduction efficiency of the active ingredient such as a nucleic acid. Phenylalanine can also be used in place of leucine.

The compounding ratio of the hydrophobic amino acid to the active ingredient such as a nucleic acid is not particularly limited, and is appropriately set as long as the dispersibility and the like of the nucleic acid can be improved. For example, the compounding ratio can be 5 parts by mass or more and 100 parts by mass or less of the hydrophobic amino acid to 1 part by mass of the nucleic acid. The compounding ratio is more preferably 5 parts by mass or more and 50 parts by mass or less of the hydrophobic amino acid. For example, the compounding ratio is 4 parts by mass or more and 24 parts by mass or less of the hydrophobic amino acid, for example, 6 parts by mass or more and 19 parts by mass or less of the hydrophobic amino acid, for example, 9 parts by mass or more and 19 parts by mass or less of the hydrophobic amino acid, and for example, 9 parts by mass or more and 24 parts by mass or less of the hydrophobic amino acid.

As an example of the composition of the present powder medicine, the present powder medicine can contain hyaluronic acid having a weight average molecular weight of 30,000 or more and 70,000 or less, preferably having a weight average molecular weight of 40,000 or more and 60,000 or less, or a salt thereof, and a hydrophobic amino acid such as phenylalanine as excipients that are components other than the active ingredient. With this composition, both the biological activity of the nucleic acid as an active ingredient (gene expression, suppression of gene expression and the like) and the inhalation performances of the present powder medicine are excellently obtained. Further, the present powder medicine can contain, for example, 40% by mass or more and 90% by mass or less, for example, 50% by mass or more and 90% by mass or less, 60% by mass or more and 90% by mass or less, and 60% by mass or more and 85% by mass or less of the hyaluronic acid or a salt thereof based on the total mass of these two components as an excipient. The content of hydrophobic amino acids such as phenylalanine can be the remained percentage. By such a percentage, both the biological activity of the nucleic acid and the inhalation performances can be easily obtained.

(Cationic carrier)

When the present powder medicine contains a nucleic acid, the present powder medicine suitably does not contain cationic carriers. Cationic carriers are generally useful for introducing a nucleic acid and the like into a cell. However, cationic carriers may exhibit cytotoxicity and the like even if the cationic carriers are non-viral cationic carriers such as cationic polymers. Thus, the present powder medicine preferably does not contain cationic carriers. Examples of such cationic carriers include, but are not limited to, a cationic polymer having a cationic group and a cationic lipid. Examples of the cationic polymer include polysaccharides having a cationic group, polypeptides having a cationic group in the side chain, and artificial polymers having a cationic group and salts thereof.

Examples of the cationic lipid (including cationic cholesterol derivatives) of such cationic carriers include DC-Chol (3β-(N-(N',N'-dimethylaminoethane)carbamoyl)cholesterol), DDAB (N,N-distearyl-N,N-dimethylammonium bromide), DMRI (N-(1,2-dimyristyloxypropa-3-yl)-N,N-dimethyl-N-hydroxyethylammonium bromide), DODAC (N,N-diorail-N,N-dimethylammonium chloride), DOGS (diheptadecylamide glycylspermidine), DOSPA (N-(1-(2,3-dioreyloxy)propyl)-N-(2-(spermine carboxamide)ethyl)-N,N-dimethylammonium trifluoroacetate), DOTAP (N-(1-(2,3-diore oiloxy)propyl)-N,N,N-trimethylammonium chloride), DOTMA (N-(1-(2,3-diorailoxy)propyl)-N,N,N-trimethylammonium chloride), and combinations thereof.

As described above, the present powder medicine can contain an excipient in addition to the active ingredient. Examples of the aspect of the excipient include at least one of leucine, trehalose, and mannitol, an anionic component, and further a hydrophobic amino acid as needed. Further, the present powder medicine can contain additives generally used in compositions containing DNA, RNA and the like intended for gene expression or suppression thereof.

The present powder medicine can contain an active ingredient. The content of the active ingredient is not particularly limited, and can be, for example, about 0.2% or more and 15% or less based on the total mass.

The active ingredient is not particularly limited as long as it can be used in the spray freeze-drying method described later. Generally, it is an organic compound and includes, for example, a nucleic acid.

The nucleic acid can include a natural nucleic acid that is a polymer of a naturally occurring deoxyribocreotide and/or ribonucleotide and an unnatural nucleic acid that is a polymer containing a deoxyribonucleotide and/or ribonucleotide having an unnatural structure at least partially. The natural deoxyribonucleotide and ribonucleotide have a natural base. The natural base is a base in a natural DNA and RNA and includes adenine, thymine, guanine, cytosine, and uracil. The natural deoxyribonucleotide and/or ribonucleotide have a backbone in which the phosphate at the 5-position of 2-deoxyribose and/or ribose and the 3'hydroxyl group of adjacent deoxyribose and/or ribose are connected by phosphodiester bond. In the present specification, the natural nucleic acid can be a DNA, an RNA, and a chimera of a deoxyribonucleotide and a ribonucleotide (hereinafter, also referred to as DNA/RNA chimera). The DNA and RNA can each be single-stranded, or double-stranded of the same type, or a hybrid in which DNA and RNA are hybridized. Further, the DNA and RNA can each be a hybrid in which a DNA/RNA chimera is hybridized with a DNA, an RNA or a DNA/RNA chimera.

An unnatural nucleic acid refers to a nucleic acid having an unnatural structure at least partially in any of a base and a backbone (sugar moiety and phosphate moiety). As an unnatural base, various unnatural bases are known. Various backbones that replace the natural ribose-phosphate backbone are also provided. Examples thereof include a glycol nucleic acid, peptide nucleic acid, and the like having about 3 carbon atoms instead of a sugar-ribose backbone. A natural nucleic acid is an L-DNA or L-RNA. A nucleic acid at least partially having the structure of a D-DNA and D-RNA is included in the unnatural nucleic acid. An unnatural nucleic acid also includes various aspects such as a single-stranded unnatural nucleic acid, a double-stranded unnatural nucleic acid, and a hybrid unnatural nucleic acid, and a chimeric unnatural nucleic acid.

These types of an unnatural nucleic acid are generally used not as a coding or template strand that encodes a protein, but, for example, as a strand that has other functions such as interaction with a certain nucleic acid in a cell to change the function of the nucleic acid. Those are typically used for functional expression such as inhibition of expression or inhibition of a function of a target protein. Examples thereof include a nucleic acid that acts directly on a nucleic acid in vivo without gene expression, and specific examples thereof include an antisense nucleic acid, a sense nucleic acid, a shRNA, a siRNA, a decoy nucleic acid, an aptamer, and a miRNA. These types of an unnatural nucleic acid are often an oligonucleotide in which about ten to several tens of nucleotides are polymerized.

In the present composition, the nucleic acid is preferably in a state of a naked nucleic acid. A naked nucleic acid is, that is, a nucleic acid that is naked. More specific examples thereof include a nucleic acid construct (non-viral vector) obtained by using a plasmid when gene expression is intended. Examples thereof include a non-viral vector such as a plasmid DNA, an antisense nucleic acid (antisense DNA or antisense RNA), a shRNA, a siRNA, a decoy nucleic acid, an aptamer, and a microRNA when suppression of gene expression is intended. The naked nucleic acid can be a nucleic acid containing a nucleic acid element for therapeutic purposes as a main component or consisting only of the nucleic acid element and not containing a nucleic acid element as a vehicle only for introducing the nucleic acid into a cell.

The form of the naked nucleic acid is not particularly limited, and can be linear, circular (ring-closed or ring-opened), or supercoiled. A form according to the purpose can be appropriately provided. The naked nucleic acid preferably does not have a viral carrier having a virus-derived element or a cationic non-viral carrier such as a liposome and a cationic polymer. This is because a viral carrier has a risk, and such a non-viral carrier is not always sufficient in terms of cytotoxicity, targeting performance, and expression efficiency.

The present powder medicine is a solid phase that has an appearance of a powder by itself by being dried, and contains a nucleic acid as an active ingredient in a part of spherical particles that constitute the powder. In the present powder medicine, the nucleic acid is in a state of a crystal or noncrystal and can be in a state forming a solid phase.

The present powder medicine can be preferably produced by a freeze-drying method, and can be more preferably produced by a spray freeze-drying method. By employing such a method of production, the present powder medicine which is hollow porous spherical particles can be easily obtained.

Thus, according to the present specification, a method of manufacturing an inhalation powder medicine containing an active ingredient, including the step of: drying a liquid containing one or more selected from the group consisting of leucine, mannitol, and trehalose as an excipient, and an active ingredient by a spray freeze-drying method is provided.

Further, according to the present specification, a drying step of spray freeze-drying a liquid in which an active ingredient and at least one excipient are dissolved so that properties of the spherical particles of the present powder medicine described above, that is, porous hollow spherical particles that are capable of being dispersed and crushed into smaller particles by inspiration and capable of swelling when the porous hollow spherical particles absorb moisture, wherein the smaller particles are also capable of swelling when the smaller particles absorb moisture, are obtained can be performed. The excipient and other conditions for obtaining such spherical particles are disclosed in the present specification.

For such a drying step, a step of selecting the excipient and preparing the liquid so that OE (%) = recovery amount on and after Throat (mg)/total recovery amount (mg) $\times$ 100 is 80% or more in inhalation performance evaluation by Andersen Cascade Impactor (ACI) of the spherical particles can be further included. Further, also, the excipient can be selected and the liquid can be prepared so that an FPF5 (%) is 30% or more in inhalation performance evaluation by ACI of the spherical particles. Further, the excipient can be selected and the liquid can be prepared so that spherical particles having a peak of a recovery percentage in any one of Filter and Stages 2 to 4 in inhalation performance evaluation by ACI of the spherical particles are obtained.

(Method of evaluating inhalation powder medicine)

According to the present specification, a method of evaluating an inhalation powder medicine is also provided. That is, a method of evaluating an inhalation powder medicine is provided, including the step of: obtaining, by calculating under the conditions described above, a first aerodynamic mass median diameter calculated in inhalation performance evaluation by ACI and a second aerodynamic mass median diameter smaller than the first aerodynamic mass median diameter. According to such a method, the reachability, which is a property of the inhalation powder medicine, can be evaluated, and in addition, the disintegration property can be also evaluated.

Further, according to the present specification, there is also provided a method of obtaining a ratio (mass) of a powder having the second aerodynamic mass median diameter based on a total powder. By obtaining such a ratio (disintegration ratio), the reachability and disintegration property of the inhalation powder medicine can also be evaluated. The disintegration ratio can be obtained together with the first and second aerodynamic mass median diameters described above.

According to the present specification, there is also provided a method of measuring at least a mass change rate at 70% RH and a mass change rate at 95% RH when RH is changed from 50% to 95% at 37°C in dynamic vapor sorption measurement. According to the method of measurement, the anti-hygroscopic property, hygroscopic property, and swelling property of the inhalation powder medicine can be evaluated.

(Use of present powder medicine)

When the active ingredient is a nucleic acid, the present powder medicine can be used for introducing a nucleic acid into a cell. Further, the present powder medicine is intended to introduce a nucleic acid into a cell for various effects by the nucleic acid such as gene expression (protein synthesis) and suppression of gene expression.

The nucleic acid contained in the present powder medicine can take various aspects according to the purpose of the present powder medicine. For example, when the nucleic acid contains a coding region encoding a protein or the like, the nucleic acid can also contain an expression control region such as a promoter and a terminator to express the protein. Examples of such a nucleic acid include an expression cassette, a plasmid vector containing an expression cassette, and an artificial chromosome. Control regions such as a promoter and a terminator and other elements can be appropriately selected and used by those skilled in the art as necessary. A plasmid vector or an artificial chromosome is appropriately selected considering the type of the cell for introduction, the size of the nucleic acid to be introduced and the like.

For example, when the expression of a gene is suppressed, as described above, examples of aspects of the nucleic acid include a sense nucleic acid, an antisense nucleic acid (DNA and RNA and the like), a shRNA, a siRNA, a miRNA, a decoy nucleic acid, and an aptamer. The nucleic acid can be a DNA formed by transcription of such an RNA or the like.

The cell to which the present powder medicine is applied is not particularly limited, and is preferably an animal cell or a microorganism cell. Examples of the animal cell include a mammalian cell including a human cell and various non-mammalian cells. Examples of the microorganism include, but are not limited to, yeast, bacteria, and fungi.

The present powder medicine can be suitably used for gene therapy for humans and animals, nucleic acid medicine, immunotherapy, embryo production, and various gene-related studies. That is, the present powder medicine can be used for, in addition to so-called in vivo gene therapy, ex vivo gene therapy. In particular, the present powder medicine is useful as a powder for preventing or treating diseases in which the action on genes by inhalation through the nasal cavity and oral cavity is effective, such as tumors in the bronchi and lungs.

The present powder medicine can be a composition for supplying to a cell substantially without an aqueous medium. "Substantially without an aqueous medium" means that the present powder medicine is applied to a cell without being dissolved or dispersed in a water-based medium (referred to as an aqueous medium in the present specification) such as a buffer. Dissolution of nucleic acids and the like in the water (moisture) existing in the place to which the present powder medicine is applied does not contradict "substantially without an aqueous medium".

The present powder medicine containing a nucleic acid as a solid substance is suitably applied in a state where the nucleic acid as a solid substance is maintained as it is, and more preferably, a solid phase powder medicine is applied to a cell in vivo. Presumably, by application of the present powder medicine or the present solid phase powder containing a nucleic acid as a solid substance to a cell, an environment advantageous for nucleic acid introduction is formed on the cell surface. Presumably, for example, the present powder medicine having such an aspect acts via the water on the cell surface existing as a gas-liquid interface in the living body, and the nucleic acid is taken up into the cell.

In animals including a human, the nucleic acid as a solid substance of the present powder medicine can reach a target site of organs that can be reached from the outside non-invasively or approximately non-invasively using a catheter or the like, for example, the inner surface (mucosa) of a nasal cavity, an eye, an oral cavity, a respiratory tract, lungs, a stomach, a duodenum, a small intestine, a large intestine, a rectum, a bladder, a vagina, an uterus, a heart, a blood vessel and the like by injection of the present powder medicine through an appropriate gas. For example, the supply of a powder preparation or the like to the pulmonary mucosa and nasal mucosa is well known as an inhalation method or the like. The present powder medicine can be directly supplied to the inside of an animal, for example, a subcutaneous part, a muscle, an abdomen, and a lesion such as a tumor by laparotomy, incision and the like. For application of the present powder medicine, a method of transplanting to the inside, the surface, or the vicinity of the target tissue can be employed. The present powder medicine can be held with being carried on the surface of a gel-like substance, a porous body such as a sponge, and a non-woven fabric.

Thus, when the present powder medicine containing a nucleic acid as a solid substance is supplied to a target site or a cell, it is supplied to the target site in a high concentration without being diluted with an aqueous medium unlike before, and can be hold at the site continuously. That is, the present powder medicine is essentially capable of reaching the target cell at a high concentration. Presumably, as a result, high uptake capability and function expression by a nucleic acid are possible.

The present powder medicine is sufficiently effective even when the present powder medicine is dissolved before use. For example, a reconstituted product of the present powder medicine prepared by suspending or dissolving the present powder medicine in an aqueous medium such as water, physiological saline, a buffer, a glucose solution, and a medium solution before use can be applied. For reconstitution, the present powder medicine is suspended in or diluted with, for example, a solvent 100 to 10000 times (weight ratio) of the nucleic acid. Because amounts and types of solvents different from those before freeze-drying can be used, relatively high-concentration suspensions and solutions (for example, a solution containing 1 mg of DNA in 1 ml), which were conventionally difficult to prepare, can be easily prepared.

The present powder medicine can be in a state in which the nucleic acid is added as a solid substance in a non-aqueous medium before use. For example, the present powder medicine can be suspended in a non-aqueous medium before use. For example, because amounts and types of solvents different from those before freeze-drying can be used, a nucleic acid can be applied based on a non-aqueous medium, which was conventionally difficult.

As described above, the present powder medicine dissolved or suspended in a suitable liquid medium can be used for any method usually used for introducing a nucleic acid or its derivative into a living cell.

The amount of the present powder medicine applied to a cell varies depending on the introduction method, the type of the disease, the purpose and the like described above. For example, the amount of the nucleic acid varies greatly depending on the administration site, and can be, for example, 5 to 1000 $\mu g/cm^3$ tumor for local administration to a tumor, for example, 0.1 $\mu g$ to 100 mg/organ for administration to an organ such as a bladder, and, for example, 0.1 ng to 10 mg /Kg/body weight for systemic administration

Examples

Hereinafter, Examples as specific examples will be described to more specifically describe the disclosure of the present specification. The following Examples are intended to describe the disclosure of the present specification and are not intended to limit its scope.

Example 1

(Preparation of inhalation powder medicine)

In this Example, the fluorescent dye sodium fluorescein (FlNa) was used as a model drug, and leucine, mannitol, and trehalose shown below were used as excipients in the combinations shown in the table below.

[Chemical Formula 1] Leucine Mannitol Trehalose

[Table 2]

| Name of preparation | Composition of preparation | Leu (mg) | Man (mg) | Tre (mg) | FlNa (mg) | Total amount |
|---|---|---|---|---|---|---|
| M0T0 | FlNa1-Leu99 | 150 | | | 1.5 | 151.5 mg/7.575 mL |
| M5T0 | FlNa1-Man5-Leu94 | 142.5 | 7.5 | | | |
| M10T0 | FlNa1-Man10-Leu89 | 135 | 15 | | | |
| M0T1 | FlNa1-Tre1-Leu98 | 148.5 | | 1.5 | | |
| M5T1 | FlNa1-Tre1-Man5-Leu93 | 141 | 7.5 | | | |
| M10T1 | FlNa1-Tre1-Man10-Leu88 | 133.5 | 15 | | | |
| M0T3 | FlNa1-Tre3-Leu96 | 145.5 | | 4.5 | | |
| M5T3 | FlNa1-Tre3-Man5-Leu91 | 138 | 7.5 | | | |
| M10T3 | FlNa1-Tre3-Man10-Leu86 | 130.5 | 15 | | | |
| M0T5 | FlNa1-Tre5-Leu94 | 142 5 | | 7.5 | | |
| M5T5 | FlNa1-Tre5-Man5-Leu89 | 135 | 7.5 | | | |
| M10T5 | FlNa1-Tre5-Man10-Leu84 | 127.5 | 15 | | | |
| F0M5T1 | Tre1-Man5-Leu94 | 141 | 7.5 | 1.5 | | 150.0 mg/7.500 mL |
| Leu100 | Leu100 | 150 | | | | |
| Man100 | Man100 | | 150 | | | |
| Tre100 | Tre100 | | | 150 | | |
| FlNa100 | FlNa100 | | | | 150 | |

Type of inhalation powder medicine and excipient (composition of mannitol and trehalose)

| Man \ Tre | 0 | 1 | 3 | 5 |
|---|---|---|---|---|
| 10 | M10T0 | M10T1 | M10T3 | M10T5 |
| 5 | M5T0 | M5T1 | M5T3 | M5T5 |
| 0 | M0T0 | M0T1 | M0T3 | M0T5 |

Powder fine particles were prepared by SFD (spray freeze-drying method). The SFD method consists of two steps, a spraying step and a freeze-drying step. First, using a two-fluid spray nozzle attached to a spray dryer (SD-1000, EYELA), a sample solution was sprayed into liquid nitrogen (500 mL) 15 cm below the tip of the nozzle at 150 kPa to be rapidly frozen. The sample solution was delivered at 5 mL/min and spraying was continued for 1.5 min. The obtained ice droplet was placed in a square dry chamber (DRC-1000 EYELA) connected to a freeze-dryer (FDU-210 EYELA), and dried under vacuum conditions for 24 hours to prepare a desired preparation.

SFD method operating conditions

[Table 3]

| | |
|---|---|
| Spray air pressure | 150 kPa |
| Sample solution flow rate | 5 mL/min |
| Spray nozzle diameter | 0.4 mm |
| Spray height | 15 cm |
| Final vacuum degree | ≤ 5 Pa |
| Final shelf temperature | 10°C |

Example 2

(Evaluation of particle shape of powder medicine by scanning electron microscope)

The particle shape of the prepared powder fine particles was observed with a scanning electron microscope (SEM: JSM-IT100LA, JEOL Ltd.). Spraying was performed using the powder fine particle addition device for dispersion addition shown in Fig. 2. As a spraying method, 0.25 mL of air was compressed in a 1 mL syringe (TERUMO) connected to a 100 μL chip filled with a small amount of the prepared powder preparation via a three-way activity, and the three-way stopcock was opened. After dispersing and adding powder fine particles on a sample mount to which black double-sided tape was attached, platinum coating (JFC-1600, JEOL Ltd.) was performed under the conditions of 30 mV and 90 sec, and SEM observation was performed. The results are shown in Fig. 2.

As shown in Fig. 2, hollow porous spherical particles were obtained regardless of the mixing ratio of excipients. Hollow porous particles were not observed for trehalose alone and sodium fluorescein alone.

Example 3

(Evaluation of particle size distribution of powder medicine by laser diffraction/scattering particle size distribution measuring instrument (LMS))

For the geometric particle size and particle size distribution of the preparation prepared in Example 1, a laser diffraction/scattering type particle size distribution measuring instrument (LMS: LMS-2000e, SEISHIN ENTERPRISE Co., Ltd.) was used. The measurement was performed by dry one-shot measurement method, and the air supply pressure was set to 0.4 MPa. A 50% particle size ($D_{50}$) was calculated from the obtained cumulative particle size distribution, and the particle size distribution was evaluated. The results are shown in Fig.3. The calculated $D_{50}$ is also shown in Fig. 3.

As shown in Fig. 3, a single particle size distribution of the prepared powder medicines was observed regardless of the mixing ratio. For some preparations (M0T0, M5T0, M0T1, M5T1, M10T3, M5T3, and M0T5), preparations around 100 to 1000 μm were observed. Similar values of $D_{50}$ of around 10 μm were calculated for all preparations.

Example 4

(Evaluation of inhalation performances of powder medicine by ACI)

Inhalation performance evaluation was performed using ACI (Low volume air sampler, Andersen type, AN-200 type, SIBATA SCIENTIFIC TECHNOLOGY LTD.) to obtain detailed data on inhalation performances.

The evaluation method was as follows: about 1.0 mg of a sample was filled in No. 2 HPMC capsule (Qualicaps Co., Ltd.), and suction was performed at a flow rate (PFR) of 28.3 L/min by Rotary Bebicon, Hitachi, Ltd. (Bebicon, 200RC-20C5, Hitachi Industrial Equipment Systems Co., Ltd.). The suction time was 10 sec. As the inhalation device, Single, Dual, and Reverse with different inhalation resistance of Jethaler (Jethaler (registered trademark), Hitachi Automotive Systems Measurement Ltd.) were used.

(Calculation of recovery percentage of powder medicine)

After dissolving the sample deposited on Device, Capsule, Throat, Filter, and each Stage in 50 ml or 10 ml of phosphate buffer (PBS), the concentration of FINa was quantified by a multi-plate reader (Enspire, PerkinElmer Co., Ltd.) (excitation wavelength: 490 nm, fluorescence wavelength: 515 nm) to calculate the recovery amount and recovery percentage of each part. Dilution and quantification were performed as needed. The recovery percentage for each Stage is shown in Fig. 4.

(Calculation of each index of powder medicine)

From the results of this experiment, OE, FPF stage3, FPF stages, and OE $\times$ FPF stages were calculated from the above Formulas (1) to (4). These indexes for various preparations are shown in Fig.5.

(Calculation of aerodynamic mass median diameter of powder medicine)

The powder medicine prepared in Example 1 tended to have a curved lognormal plot, and thus it was presumed that a part of the powder disintegrated, and a powder having a large particle size (MMAD and GSD are defined as $MMAD_c$ and $GSD_c$) and a powder having a small particle size ($MMAD_f$ and $GSD_f$) were produced in a ratio of (1-R) : R. Thus, from the eight measurement points (cumulative values of recovery percentage) obtained by ACI analysis, the following values A to D were determined for each Stage using Excel functions, and R, $MMAD_c$, $GSD_c$, $MMAD_f$, and $GSD_f$ that minimize E were determined by the solver function of Excel. These results are shown in Fig. 6. An example of the analysis results of the powder before and after the particle disintegration is also shown in Fig. 6.

A = Random variable obtained by converting the cumulative value (%) of the recovery percentage for each Stage by the NORM.S.INV function.

B = Cumulative value of recovery percentage of powder of $MMAD_c$ and $GSD_c$ calculated by NORM.DIST function

C = Cumulative value of recovery percentage of powder of $MMAD_f$ and $GSD_f$ calculated by NORM.DIST function

Random variable obtained by converting the value of $D = B \times (1-R) + C \times R$ by the NORM.S.INV function

E = Total value of $(D-A)^2$ determined for each Stage

As shown in Fig. 4 and Fig. 5, the deposition of the preparation on the Filter was the highest and was 20% or more. Stage 3 had the highest recovery percentage among Stages for all preparations. As shown in Fig. 5, good indexes (OE, FPF) tended to be obtained in particular, in the preparation containing mannitol.

As shown in Fig. 6, the disintegration ratio R analyzed by the solver function was about 45% when the Man content was 0%, and was about 50 to 60% when the Man content was 5 to 10%. The disintegration ratio R increased with the addition of Man. Meanwhile, the addition of Man reduced $MMAD_c$ and $MMAD_f$ (Man 0%: about 4.3 μm, Man 5 to 10%: 3.5 to 4.0 μm) (Man 0%: about 0.35 μm, Man 5 to 10%: 0.2 μm level). Thus, it was found that mannitol greatly contributes to the disintegration property.

Example 5

(Evaluation of anti-hygroscopic property and hygroscopic property of powder medicine)

For each SFD preparation prepared in Example 1 and each raw powder (Leu, Man, Tre, FINa) in compositions, using a Dynamic Vapor Sorption measuring instrument (DVS: Dynamic Vapor Sorption; DVS advantage, Surface Measurement Systems), anti-hygroscopic property and hygroscopic growth from inhalation to the respiratory tract and deep lungs in high humidity were evaluated.

By a DVS, the mass change of a sample filled on one side of the balance-type measuring unit due to the adsorption and desorption of water in a set temperature and humidity environment can be monitored on a second scale. The measurement conditions for evaluation are shown in the table below. Regarding the setting of conditions for moisture absorption growth evaluation, to reproduce the temperature and humidity environment changes until the powder fine particles reach the deep lungs, the environment before inhalation was set to "temperature: 37°C, relative humidity (RH): 50% (absolute humidity: 6.903 g/m$^3$)" and the lung environment after inhalation was set to "temperature: 37°C, 95% RH (absolute humidity: 41.62 g/m$^3$)". The results are shown in Fig. 7.

DVS measurement conditions

[Table 4]

| | Anti-hygroscopic property and hygroscopic growth in lung environment |
|---|---|
| Temperature (°C) | 37 |
| Starting humidity (% RH) | 50 |
| End humidity (% RH) | 95 |
| dm/dt (%/min) | 0.005 |
| Measuring humidity interval (% RH) | 10 (50-70% RH), 5 (70-95% RH) |

As shown in Fig. 7, the comparison between SFD preparations showed that the SFD preparation having a higher proportion of Man and Tre absorbed moisture with increasing relative humidity, and had a higher mass change rate. The higher the proportion of Leu, the higher the anti-hygroscopic property even at high humidity. The mass change rate of the SFD preparation was higher than the mass change rate of Leu, Man and Tre alone when the raw powders (about 0.08, 0.29, 22%) and the SFD preparations (about 0.14, 2.40, 41%) were compared.

Example 6

(Long-term storage test of powder medicine (anti-hygroscopic property/hygroscopic property))

In the present Example, using plasmid DNA encoding firefly luciferase (for inhalation performances, sodium fluorescein (FINa) that is a fluorescent dye was used) as a model drug, and L-leucine (hereinafter also referred to as Leu) and hyaluronic acid (sodium salt, weight average molecular weight: 50,000, hereinafter also referred to as LHA) as excipients, a liquid for spray freeze-drying was prepared based on the following composition, and spray freeze-dried.

[1] Sample 1 (pDNA/LHA/Leu)

Aqueous solution containing 1 mg of pDNA, 12.5 mg of LHA, and 36.5 mg of Leu (50 mg in total)

[2] Sample 2 (pDNA/LHA),

Aqueous solution containing 1 mg of pDNA and 49 mg of LHA (50 mg in total)

The spray freeze-drying was performed according to Example 1 at a spray air pressure of 150 kPa, a sample solution flow rate of 5 ml/min, a spray nozzle diameter of 0.4 mm, a drying time of 24 hours, a final vacuum degree of 5 Pa or less, and a final shelf temperature of 10°C. The obtained powder preparations were stored for up to 12 months under the three conditions of 5°C/dry (silica gel), 25°C/dry (silica gel), and 25°C/75% RH to evaluate SEM and both inhalation performances and gene expression.

(SEM observation)

In both Sample 1 and Sample 2, though the hollow porous structure was maintained even after 12 months under dry conditions, Sample 1 absorbed moisture one month after the start of storage, Sample 2 absorbed moisture immediately after storage through one week after storage, and after that, the hollow porous structure was not substantially maintained under humidified conditions (75% RH). According to SEM observation, the anti-hygroscopic property of Sample 1 was superior to that of Sample 2.

(Inhalation performances)

The inhalation performances were evaluated according to Example 4. A powder medicine containing FINa was used for inhalation performance evaluation. The results are shown in Figs. 9A and 9B. As shown in Fig. 9A, in Sample 1, though a significant decrease in inhalation performances was not observed under dry conditions, FPF3 decreased and MMAD increased in and after 4 months under the humidified condition. This indicates that the initial spherical particles were agglomerated due to moisture absorption. As shown in Fig. 9B, in Sample 2, though the inhalation performances did not change with the storage period under dry conditions, the FPF3 was low and the MMAD was as large as about 5 to 8 μm. Under the humidified condition, measurement was impossible due to moisture absorption.

(Gene expression)

The powder medicines of Samples 1 and 2 were administered to the lungs of mice to evaluate the gene expression effect. Administration to mice and the evaluation were performed as follows.

(1) Intrapulmonary administration to mice

As a pretreatment, the anterior teeth of a female ICR mouse (5 weeks old) were placed on a self-made fixing plate under anesthesia of pentobarbital (50 mg/kg, i.p.) so that the chest would be vertical. The mouth of the mouse was opened and the tongue was pulled out with tweezers while locally shining light on the chest using a light (MegaLight 100 (trademark), SCHOTT Japan Corporation). After finding the trachea that appeared as a white hole in the mouth, a mouse intrapulmonary cannula (PE-60 polyethylene tube having a total length of 4.0 cm) attached to the spray tip of an intubation aid (Liquid MicroSprayer (trade name, PennCentury, Inc.)) was inserted into the trachea 3.0 cm.

After pulling out only the intubation aid while leaving the cannula in the trachea, the passage of exhalation from the cannula was confirmed, then the tip of the device for intrapulmonary administration was inserted into the cannula, and 0.25 mL of compressed air was released in accordance with the inspiration of the mouse to administer 0.5 mg (10 μg as pDNA) of each powder medicine filled in the tip in advance to the lungs.

(2) Evaluation of gene expression effect in lungs

Luminescence based on luciferase activity was evaluated by detection and analysis using IVIS (trademark). The luciferin adjusted to 30 mg/mL using PBS and stored at -80°C was used for measurement. The mouse was anesthetized with Isoflurane (Isoflu, trademark, Abbott Laboratories), and luciferin, a luminescent substrate (30 mg/mL, 0.05 mL/mouse; 300 mg/kg) was nasally administered 6, 12, 24, and 48 hours after intrapulmonary administration to the mouse. Ten minutes after administration of luciferin, luminescence was detected at an exposure time of 1 minute under isoflurane anesthesia. A region of interest (ROI; length: 1 cm, width: 3 cm) corresponding to the lungs was produced, its luminescence intensity (Total Flux (photon/sec)) was determined as the gene expression level, and the gene expression level-time pattern was analyzed. From the obtained gene expression level-time pattern, the area under the curve (AUC) of the gene expression level-time and the maximum gene expression level (Luc (max)) were each determined. The results are shown in Fig 10.

As shown in Fig. 10, in Sample 1 and Sample 2, the initial gene expression was generally maintained even after 12 months under dry conditions, but it was significantly decreased at 4 months under the humidified condition. The expression level in Sample 2 was higher than that in Sample 1.

From the above, it was found that the inhalation performances and gene expression properties of the present powder medicine can be maintained under the dry condition.

Example 7

(Molecular weight of hyaluronic acid and gene expression)

In the present Example, the influence of powder medicines containing hyaluronic acid (sodium salt) of various molecular weights on gene expression was investigated.

Using hyaluronic acid having a weight average molecular weights of 2,000, 5,000, 50,000, 80,000, and 350,000 (each referred to as HA2 and the like), a solution for spray freeze-drying containing 1 mg (2% by mass) of pDNA (plasmid DNA encoding firefly luciferase), 12.5 mg (25% by mass) of each hyaluronic acid, and 36.5 mg (73% by mass) of phenylalanine, for a total of 50 mg, was prepared. For hyaluronic acid, a solution prepared to pH 7.0 ± 0.5 with NaOH was used. This solution was spray freeze-dried by the same method as in Example 6 to produce a powder medicine.

A549 cells, which are human-derived alveolar cancer cells, were cultured for 4 to 9 days at a cell seeding

number of 2 × $10^2$ cells/well (gas-liquid interfacial culture system Transwell (registered trademark)), and then a constant amount of each powder medicine prepared was added to the wells from a powder addition device filled with 0.4 to 0.6 mg of the powder medicine. After 48 hours, the cells in the wells were frozen and thawed to be destroyed, and then PicaGene was added and the fluorescence was measured with a luminometer. The results are shown in Fig. 11. As shown in Fig. 11, HA50 having a weight average molecular weight of 50,000 showed the highest gene expression.

Then, for HA50 having a weight average molecular weight of 50,000, four types of 5 ml each of a solution for spray freeze-drying containing 1 mg (2% by mass) of pDNA, 12.5 mg (25% by mass) of HA, and 36.5 mg (73% by mass) of phenylalanine, a solution for spray freeze-drying containing 1 mg (2% by mass) of pDNA, 24.5 mg (49% by mass) of HA, and 24.5 mg (49% by mass) of phenylalanine, a solution for spray freeze-drying containing 1 mg (2% by mass) of pDNA, 36.5 mg (73% by mass) of HA, and 12.5 mg (35% by mass) of phenylalanine, and a solution for spray freeze-drying containing 1 mg (2% by mass) of pDNA, 49 mg (98%) of HA, and 0 mg (0% by mass) of phenylalanine were prepared. This solution was spray freeze-dried by the same method as in Example 6 to produce a powder medicine, and gene expression was evaluated in the same manner as described above. The results are shown in Fig. 12.

As shown in Fig. 12, the composition containing 73% by mass of HA50 and 12.5% by mass of phenylalanine showed the highest gene expression effect. The above results showed that HA50 is suitable and phenylalanine is usefully used in combination from the viewpoint of gene expression. From the viewpoint of gene expression, HA50 is suitably contained in an amount of 50% by mass or more, for example, 50% by mass or more, for example, 60% by mass or more, or for example, 70% by mass or more in all excipients, and is suitably contained in an amount of, for example, 90% by mass or less, for example, 85% by mass or less, or for example, 80% by mass or less. The range defined by the combination of these lower and upper limits is also suitable. It was found that HA50 and excipients such as phenylalanine are useful in the range defined by the lower limit selected from lower limits of, for example, 10 parts by mass or more, for example, 15 parts by mass, and for example, 20 parts by mass or more of an excipient relative to 100 parts by mass of HA50 and the upper limit selected from upper limits of, for example, 40 parts by mass or less, for example, 35 parts by mass or less, and for example, 30 parts by mass or less of an excipient relative to 100 parts by mass of HA50.

Example 8

(Inhalation performances of powder medicine containing hyaluronic acid and phenylalanine)

The inhalation performances of the powder medicine containing hyaluronic acid (weight average molecular weight 50,000) and phenylalanine, which were confirmed to have good gene expression in Example 7, was investigated. In the present Example, using sodium fluorescein (FlNa), a fluorescent dye, and L-phenylalanine (hereinafter, also referred to as Phe) and hyaluronic acid (weight average molecular weight: 50,000, hereinafter also referred to as HA50) as excipients, a liquid for spray freeze-drying was prepared based on the following composition, and spray freeze-dried. HA50 was used as a solution prepared to pH 7 with NaOH.

[1] Sample 1
A solution (5 ml) containing 2% by mass of FlNa, 98% by mass of HA50, and 0% by mass of Phe (50 mg in total).
[2] Sample 2
A solution (5 ml) containing 2% by mass of FlNa, 73% by mass of HA50, and 25% by mass of Phe (50 mg in total)
[3] Sample 3
A solution (5 ml) containing 2% by mass of FlNa, 49% by mass of HA50, and 49% by mass of Phe (50 mg in total).
[4] Sample 4
A solution (5 ml) containing 2% by mass of FlNa, 25% by mass of HA50, and 73% by mass of Phe (50 mg in total)

The spray freeze-drying was performed according to Example 6 to obtain a powder medicine, and the inhalation performances were evaluated according to Example 4. The results are shown in Fig. 13. As shown in Fig. 13, the lower the % by mass of HA50 and the higher the % by mass of Phe, the higher the reachability to the lungs. All of the preparations also showed good MMAD. The lower the % by mass of HA50 and the higher the % by mass of Phe, the smaller the MMAD.

As a result of such inhalation performance evaluation, it was found that the intrapulmonary arrival percentages such as "FPF3" and "OE × FPF3" were 20% or more for all the preparations. It was also found that with the HA 25% preparation, that is, a preparation having higher Phe content, higher inhalation performances are obtained. It was also found that all the preparations had a MMAD of 1 to 6 μm, and thus are suitable as an inhalation medicine. Thus, according to the present Example, it was found that the combination of HA50 and phenylalanine is good.

The efficacy of an inhalation powder medicine containing HA50 and phenylalanine as excipients, which can be presumed from the gene expression effect in vitro shown in Fig. 12 and the results of the inhalation performances shown in Fig. 13 is shown in Fig. 14.

As shown in Fig. 14, it was found that when the content of HA50 is 40% by mass or more and 90% by mass or less, for example, 50% by mass or more and 90% by mass or less, 60% by mass or more and 90% by mass or less, 60% by mass or more and 85% by mass or less, and 60% by mass or more and 80% by mass or less based on the total mass of HA50 and phenylalanine, high efficacy as an inhalation powder medicine is expected.

## Claims

1. An inhalation powder medicine, comprising: an active ingredient in at least a part of porous hollow spherical particles that are capable of being dispersed and crushed into smaller particles by inspiration and capable of swelling when the porous hollow spherical particles absorb moisture, wherein the smaller particles are also capable of swelling when the smaller particles absorb moisture.

2. The inhalation powder medicine according to claim 1, having OE (%) = recovery amount on and after Throat (mg)/total recovery amount (mg) $\times$ 100 of 80% or more in inhalation performance evaluation by Andersen Cascade Impactor (ACI).

3. The inhalation powder medicine according to claim 1 or 2, having an FPF5 (%) of 30% or more in inhalation performance evaluation by ACI.

4. The inhalation powder medicine according to any one of claims 1 to 3, having a peak of a recovery percentage in any one of Filter and Stages 2 to 4 in inhalation performance evaluation by ACI.

5. The inhalation powder medicine according to any one of claims 1 to 4, having a first aerodynamic mass median diameter calculated in inhalation performance evaluation by ACI and a second aerodynamic mass median diameter calculated in inhalation performance evaluation by ACI larger than the first aerodynamic mass median diameter.

6. The inhalation powder medicine according to claim 5, having a percentage (mass) of a powder having the second aerodynamic mass median diameter based on a total powder of 40% or more.

7. The inhalation powder medicine according to any one of claims 1 to 6, having a mass change rate at 70% RH of 1% or less and a mass change rate at 95% RH of 5% or more when RH is changed from 50% to 95% at 37°C in dynamic vapor sorption measurement.

8. The inhalation powder medicine according to any one of claims 1 to 7, wherein the spherical particles contain leucine, mannitol, and trehalose as an excipient.

9. The inhalation powder medicine according to any one of claims 1 to 8, wherein the particles have a peak particle size in geometric particle size distribution of 1 $\mu$m or more and 100 $\mu$m or less.

10. The inhalation powder medicine according to any one of claims 1 to 9, comprising a nucleic acid as the active ingredient.

11. The inhalation powder medicine according to claim 10, wherein the nucleic acid is a naked nucleic acid.

12. The inhalation powder medicine according to claim 10 or 11, further comprising an anionic component that is an anionic polymer or a salt thereof as an excipient.

13. The inhalation powder medicine according to claim 12, wherein the anionic component is hyaluronic acid or a salt thereof.

14. The inhalation powder medicine according to claim 13, wherein the hyaluronic acid has a weight average molecular weight of 30,000 or more and 70,000 or less.

15. The inhalation powder medicine according to any one of claims 10 to 14, further comprising one or more hydrophobic amino acids as an excipient.

16. The inhalation powder medicine according to claim 15, wherein the hydrophobic amino acids are one or more selected from the group consisting of leucine, phenylalanine, and isoleucine.

**17.** The inhalation powder medicine according to claim 10 or 11, comprising hyaluronic acid having a weight average molecular weight of 30,000 or more and 70,000 or less or a salt thereof and phenylalanine as an excipient that is a component other than the active ingredient, wherein the inhalation powder medicine has a content of the hyaluronic acid or a salt thereof of 40% by mass or more and 85% by mass or less based on a total mass of these two components.

**18.** The inhalation powder medicine according to any one of claims 10 to 17, for gene expression in a mammal.

**19.** The inhalation powder medicine according to any one of claims 10 to 17, for gene suppression in a mammal.

**20.** A method of manufacturing an inhalation powder medicine, comprising:
a drying step of spray freeze-drying a liquid in which an active ingredient and at least one excipient are dissolved so that porous hollow spherical particles that are capable of being dispersed and crushed into smaller particles by inspiration and capable of swelling when the porous hollow spherical particles absorb moisture, wherein the smaller particles are also capable of swelling when the smaller particles absorb moisture, are obtained.

**21.** The method according to claim 20, further comprising the step of: selecting the at least one excipient and preparing the liquid so that OE (%) = recovery amount on and after Throat (mg)/total recovery amount (mg) × 100 is 80% or more in inhalation performance evaluation by Andersen Cascade Impactor (ACI) of the spherical particles.

**22.** The method according to claim 21, wherein the at least one excipient is selected and the liquid is prepared so that an FPF5 (%) is 30% or more in inhalation performance evaluation by ACI of the spherical particles.

**23.** The method according to any one of claims 20 to 22, wherein the at least one excipient is selected and the liquid is prepared so that spherical particles having a peak of a recovery percentage in any one of Filter and Stages 2 to 4 in inhalation performance evaluation by ACI of the spherical particles are obtained.

**24.** The method according to any one of claims 20 to 23, wherein the drying step is a step of drying a liquid containing an active ingredient by a spray freeze-drying method using leucine, mannitol, and trehalose as the at least one excipient.

**25.** A method of evaluating an inhalation powder medicine, comprising the step of:
obtaining a first aerodynamic mass median diameter calculated in inhalation performance evaluation by ACI and a second aerodynamic mass median diameter calculated in inhalation performance evaluation by ACI smaller than the first aerodynamic mass median diameter.

**26.** The method according to claim 25, wherein a percentage (mass) of a powder having the second aerodynamic mass median diameter based on a total powder is 40% or more.

**27.** A method of evaluating an inhalation powder medicine, comprising the step of:
measuring at least a mass change rate at 70% RH and a mass change rate at 95% RH when RH is changed from 50% to 95% at 37°C in dynamic vapor sorption measurement.

FIG. 1

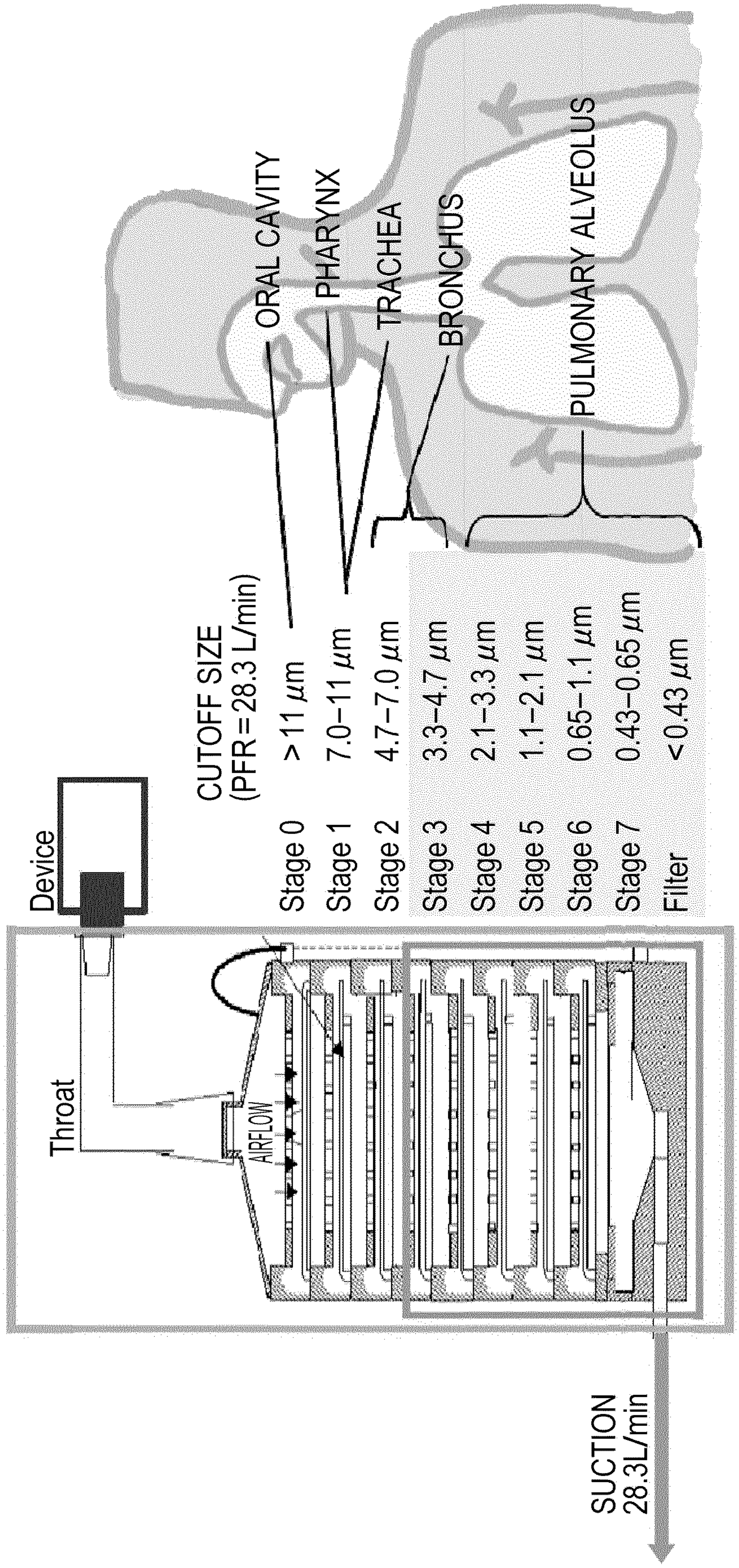
Device
Throat
AIRFLOW
SUCTION
28.3L/min
CUTOFF SIZE
(PFR = 28.3 L/min)
Stage 0 >11 μm
Stage 1 7.0–11 μm
Stage 2 4.7–7.0 μm
Stage 3 3.3–4.7 μm
Stage 4 2.1–3.3 μm
Stage 5 1.1–2.1 μm
Stage 6 0.65–1.1 μm
Stage 7 0.43–0.65 μm
Filter <0.43 μm
ORAL CAVITY
PHARYNX
TRACHEA
BRONCHUS
PULMONARY ALVEOLUS

FIG. 2

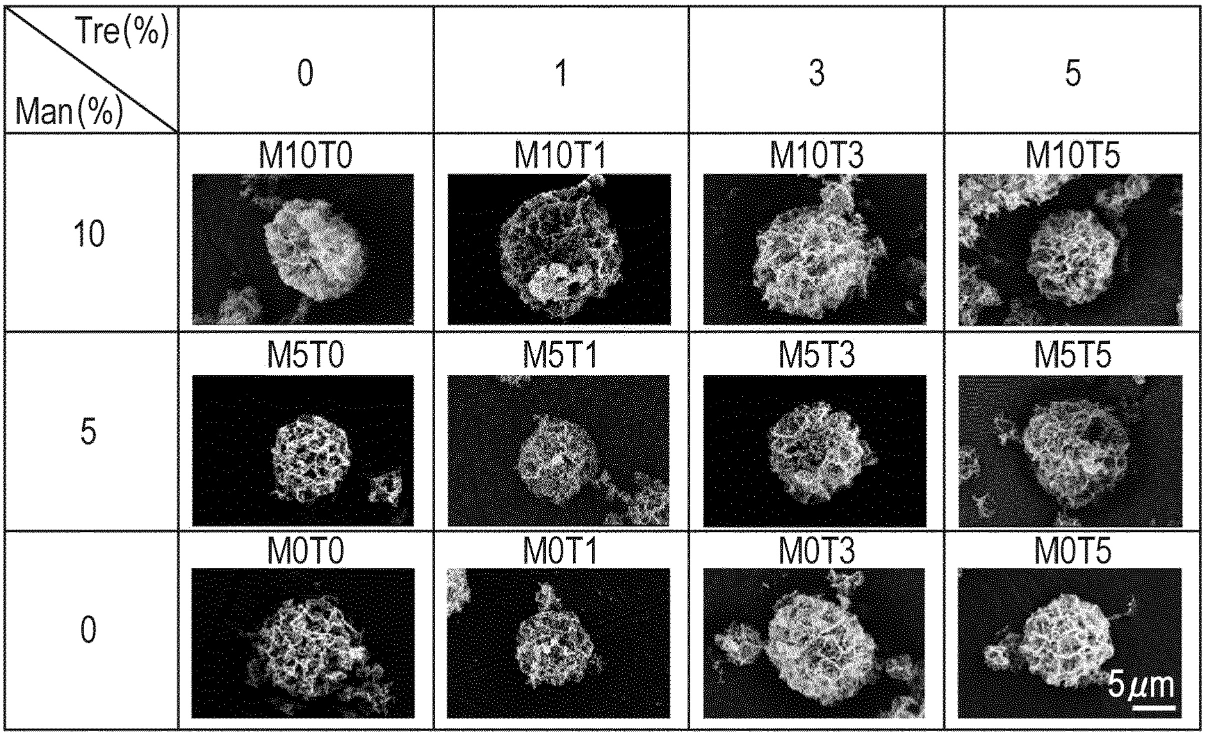


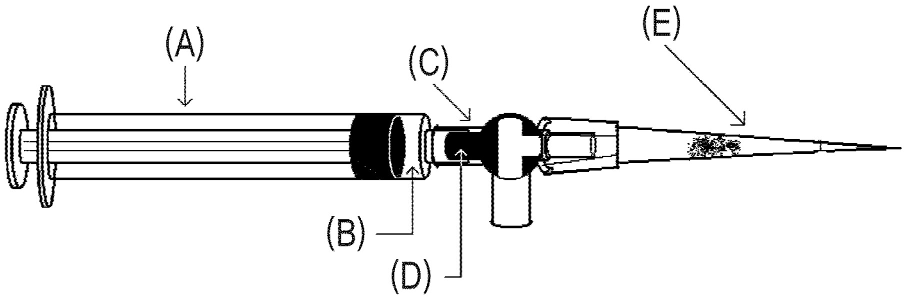


POWDER FINE PARTICLE ADDITION DEVICE
(A) 1 mL SYRINGE, (B) COMPRESSED AIR (0.25 mL), (C) THREE-WAY STOPCOCK, (D) HANDLE FOR ADMINISTRATION, (E) DISPOSABLE TIP

FIG. 3

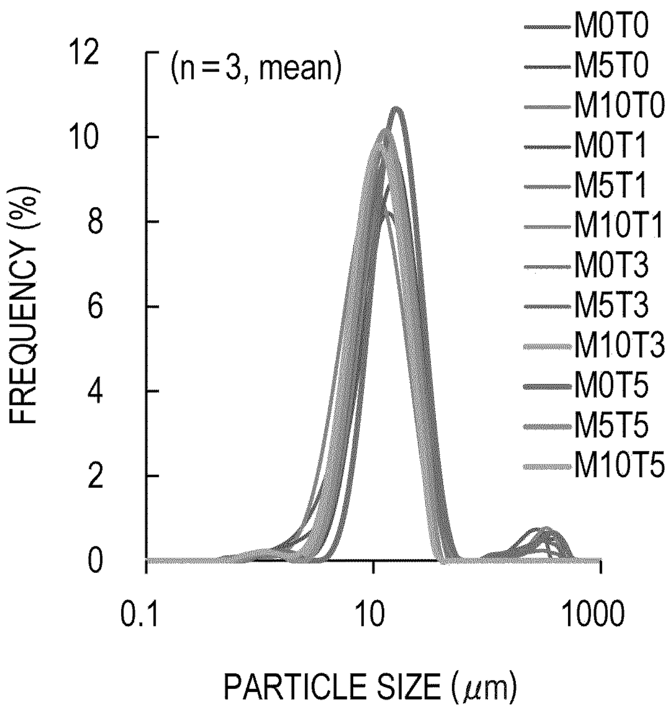


| NAME OF PREPARATION | $D_{50}$ ±S.D. |
|---|---|
| M0T0 | 11.32 ±0.073 |
| M5T0 | 13.28 ±0.045 |
| M10T0 | 8.94 ±0.310 |
| M0T1 | 13.19 ±0.187 |
| M5T1 | 11.99 ±0.308 |
| M10T1 | 12.74 ±0.051 |
| M0T3 | 11.43 ±0.019 |
| M5T3 | 12.49 ±0.062 |
| M10T3 | 10.70 ±0.032 |
| M0T5 | 14.84 ±0.039 |
| M5T5 | 11.41 ±0.078 |
| M10T5 | 9.93 ±0.019 |

FIG. 4

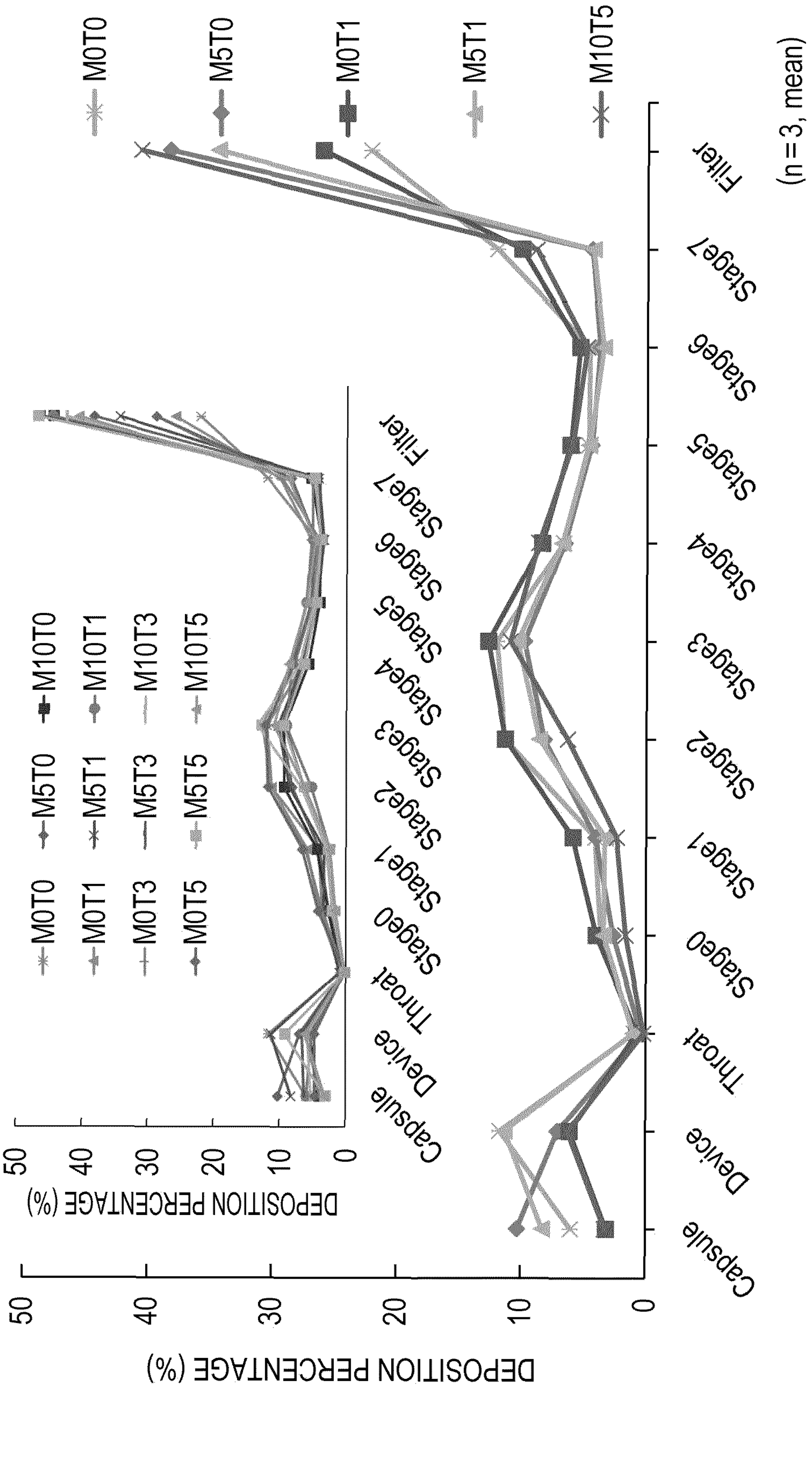

DEPOSITION PERCENTAGE (%)
50
40
30
20
10
0
Capsule
Device
Throat
Stage0
Stage1
Stage2
Stage3
Stage4
Stage5
Stage6
Stage7
Filter
M0T0
M5T0
M0T1
M5T1
M10T5
(n=3, mean)
M0T0
M0T1
M0T3
M0T5
M5T0
M5T1
M5T3
M5T5
M10T0
M10T1
M10T3
M10T5

FIG. 5

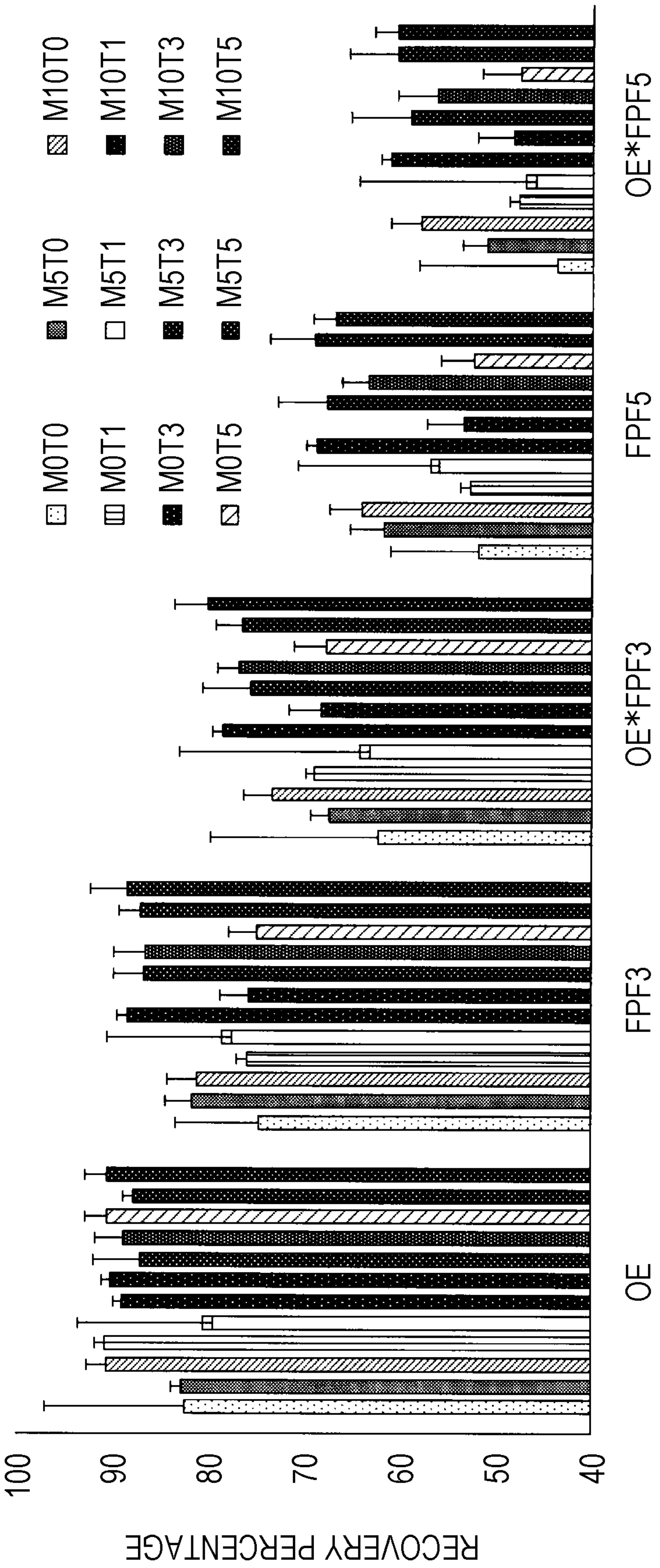
RECOVERY PERCENTAGE
100
90
80
70
60
50
40
OE
FPF3
OE*FPF3
FPF5
OE*FPF5
M0T0
M0T1
M0T3
M0T5
M5T0
M5T1
M5T3
M5T5
M10T0
M10T1
M10T3
M10T5

FIG. 6

| | MMADc | GSDc | MMADf | GSDf | R |
|---|---|---|---|---|---|
| M0T0 | 4.31 ±0.48 | 1.89 ±0.06 | 0.38 ±0.09 | 1.41 ±0.03 | 45.19 ±7.91 |
| M5T0 | 4.02 ±0.22 | 1.94 ±0.03 | 0.27 ±0.09 | 1.77 ±0.25 | 51.13 ±7.47 |
| M10T0 | 4.31 ±0.40 | 1.87 ±0.08 | 0.22 ±0.02 | 1.95 ±0.13 | 59.91 ±2.11 |
| M0T1 | 4.16 ±0.72 | 1.95 ±0.08 | 0.35 ±0.06 | 1.65 ±0.30 | 44.40 ±4.71 |
| M5T1 | 3.95 ±0.79 | 2.00 ±0.04 | 0.26 ±0.02 | 1.60 ±0.17 | 48.93 ±11.15 |
| M10T1 | 3.00 ±0.52 | 2.17 ±0.14 | 0.27 ±0.07 | 1.53 ±0.35 | 56.41 ±4.33 |
| M0T3 | 4.37 ±0.15 | 1.87 ±0.01 | 0.35 ±0.02 | 1.57 ±0.04 | 46.86 ±3.63 |
| M5T3 | 3.45 ±0.31 | 2.05 ±0.03 | 0.21 ±0.01 | 1.76 ±0.11 | 59.05 ±4.44 |
| M10T3 | 3.76 ±0.58 | 1.90 ±0.02 | 0.28 ±0.06 | 1.62 ±0.19 | 52.00 ±5.75 |
| M0T5 | 4.44 ±0.34 | 1.91 ±0.06 | 0.32 ±0.08 | 1.73 ±0.25 | 45.63 ±3.99 |
| M5T5 | 3.56 ±0.16 | 2.01 ±0.06 | 0.23 ±0.02 | 1.73 ±0.12 | 60.85 ±4.92 |
| M10T5 | 3.08 ±0.34 | 2.01 ±0.03 | 0.29 ±0.07 | 1.52 ±0.24 | 59.10 ±5.93 |

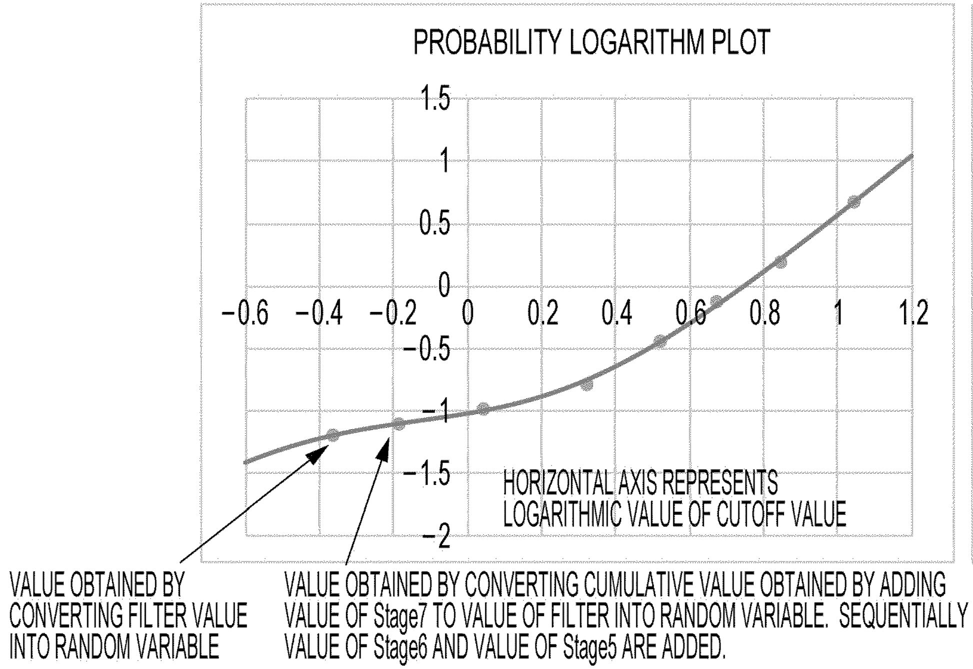


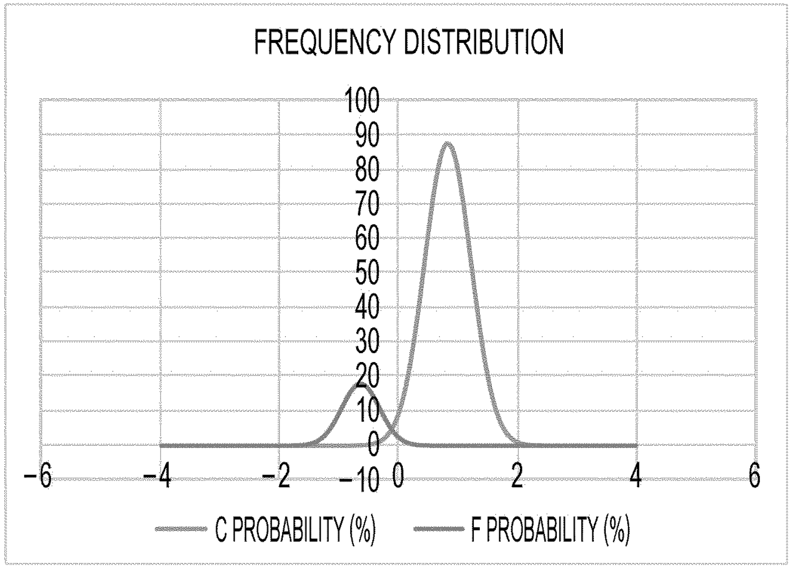

FIG. 7

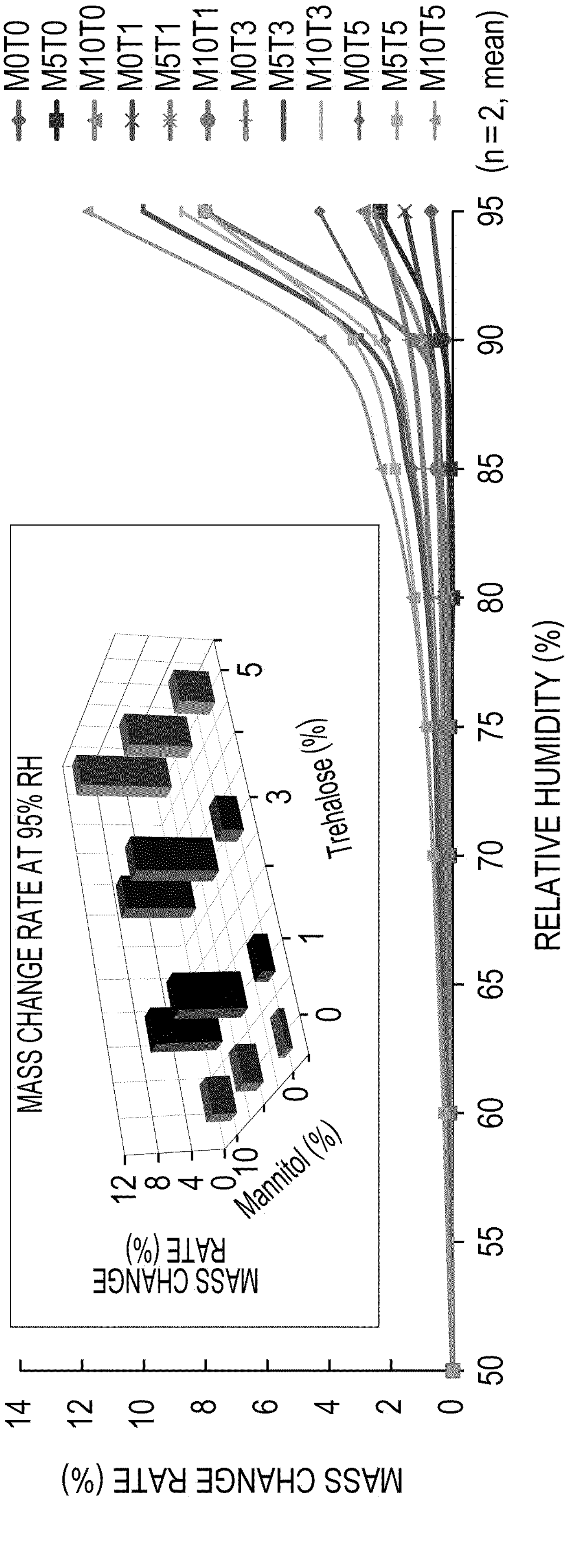
MASS CHANGE RATE (%)
14
12
10
8
6
4
2
0
50
55
60
65
70
75
80
85
90
95
RELATIVE HUMIDITY (%)
M0T0
M5T0
M10T0
M0T1
M5T1
M10T1
M0T3
M5T3
M10T3
M0T5
M5T5
M10T5
(n=2, mean)
MASS CHANGE RATE AT 95% RH
MASS CHANGE RATE (%)
12
8
4
0
10
5
0
Mannitol (%)
0
1
3
5
Trehalose (%)

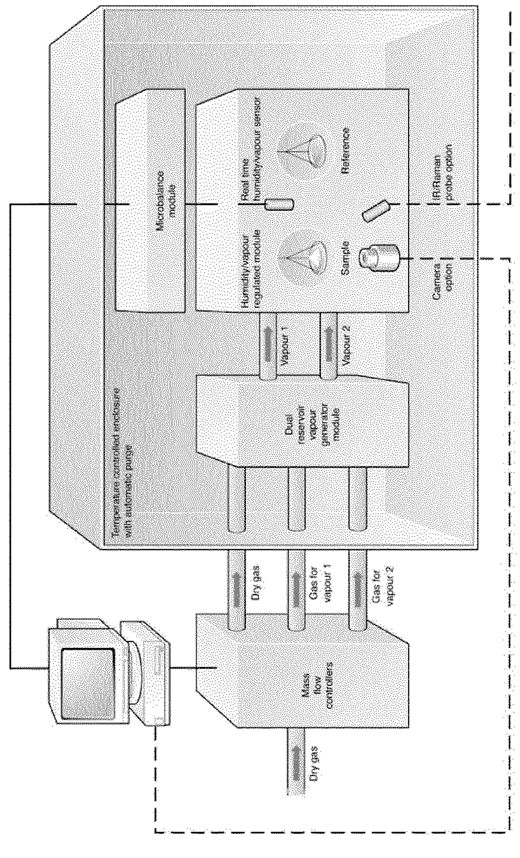
Temperature controlled enclosure with automatic purge
Microbalance module
Humidity/vapour regulated module
Real time humidity/vapour sensor
Sample
Reference
Camera option
IR/Raman probe option
Dual reservoir vapour generator module
Vapour 1
Vapour 2
Mass flow controllers
Dry gas
Gas for vapour 1
Gas for vapour 2
Dry gas

# FIG. 8

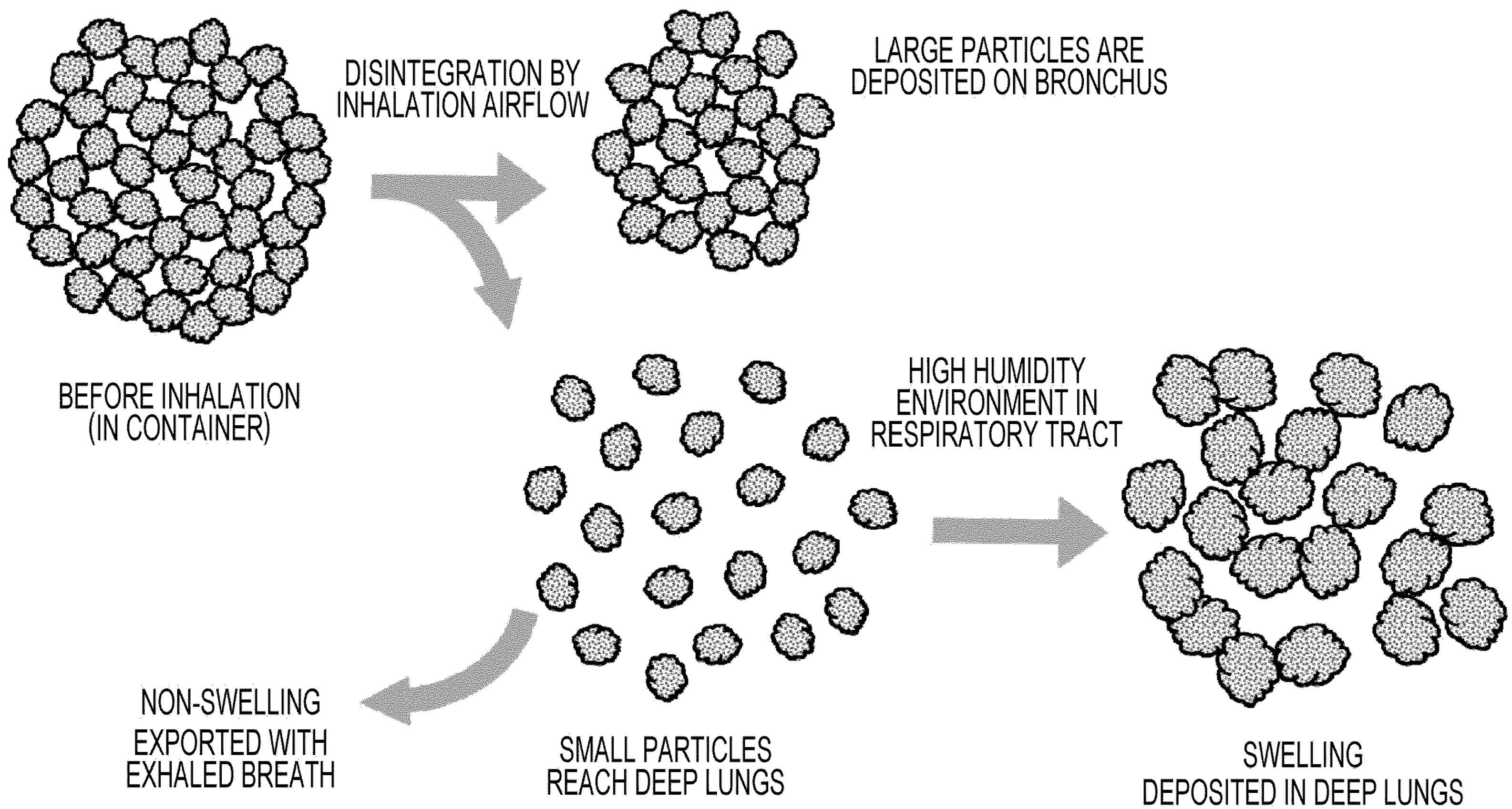
DISINTEGRATION BY INHALATION AIRFLOW
LARGE PARTICLES ARE DEPOSITED ON BRONCHUS
BEFORE INHALATION (IN CONTAINER)
HIGH HUMIDITY ENVIRONMENT IN RESPIRATORY TRACT
NON-SWELLING EXPORTED WITH EXHALED BREATH
SMALL PARTICLES REACH DEEP LUNGS
SWELLING DEPOSITED IN DEEP LUNGS

# FIG. 9A

INHALATION PERFORMANCE EVALUATION OF INHALATION POWDER MEDICINE (ACI)
SAMPLE 1: FlNa/LHA/Leu

(n=3, mean+S.D.)

*pDNA WAS REPLACED WITH FlNa

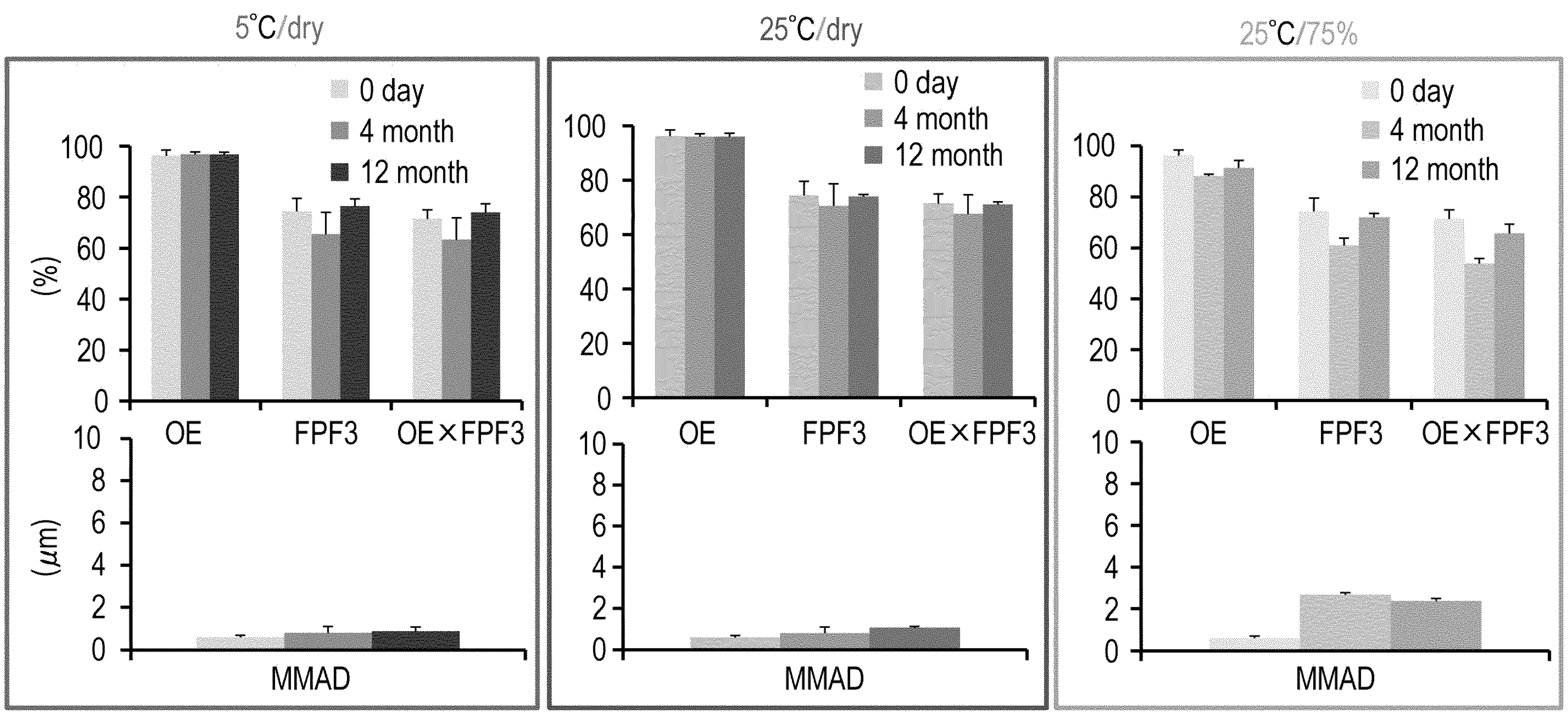

# FIG. 9B

INHALATION PERFORMANCE EVALUATION OF INHALATION POWDER MEDICINE (ACI)
SAMPLE 2: FINa/LHA

(0 day: n = 1)
(4 month,12 month: n = 3, mean+S.D.)

*pDNA WAS REPLACED WITH FINa

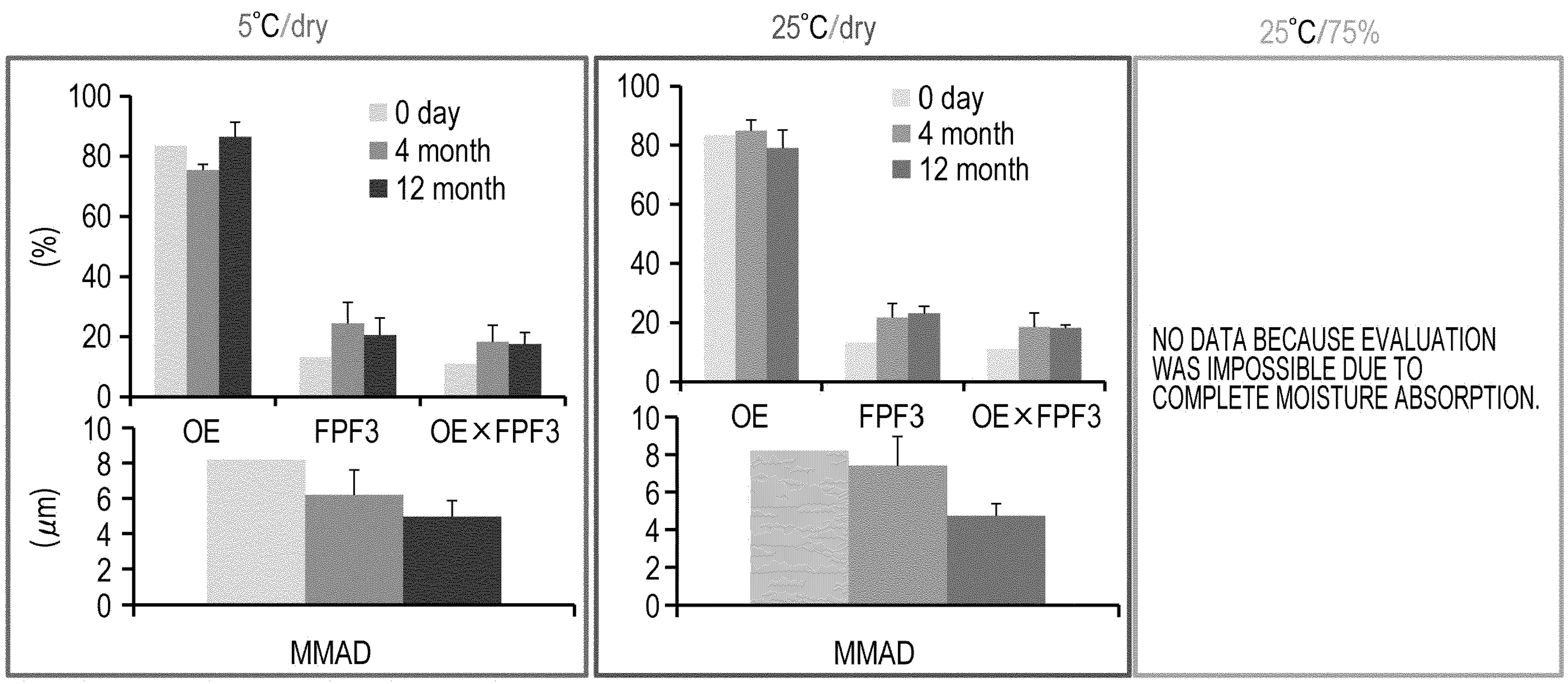

FIG. 10

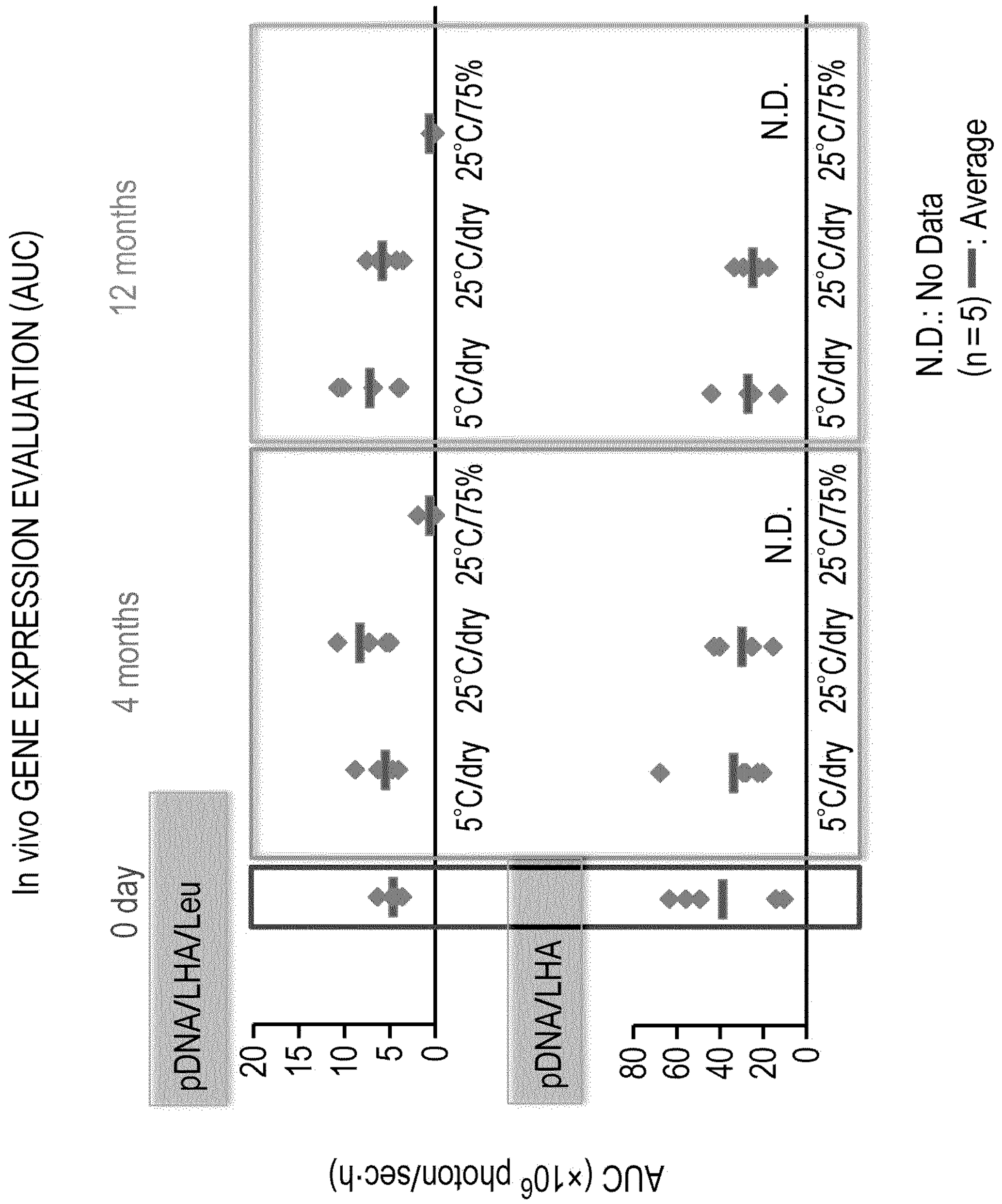

In vivo GENE EXPRESSION EVALUATION (AUC)
0 day
4 months
12 months
pDNA/LHA/Leu
pDNA/LHA
AUC (×10^6 photon/sec·h)
20
15
10
5
0
80
60
40
20
0
5°C/dry
25°C/dry
25°C/75%
N.D.
N.D.: No Data
(n=5) ━ : Average

FIG. 11

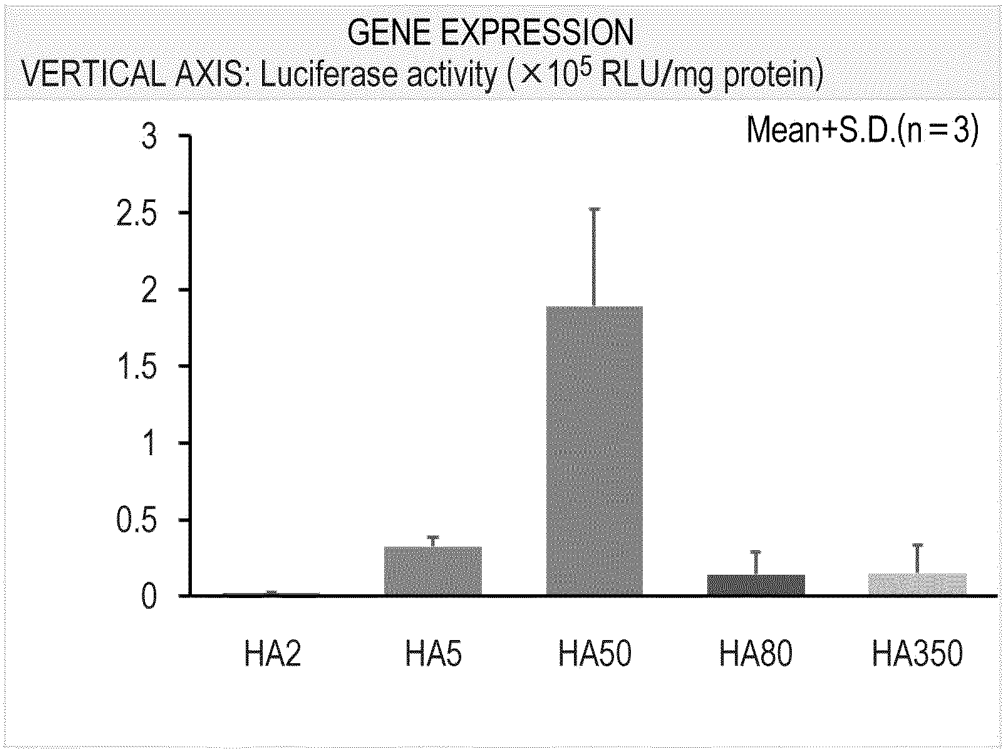
GENE EXPRESSION
VERTICAL AXIS: Luciferase activity (×10^5 RLU/mg protein)
Mean+S.D.(n=3)
3
2.5
2
1.5
1
0.5
0
HA2
HA5
HA50
HA80
HA350

FIG. 12

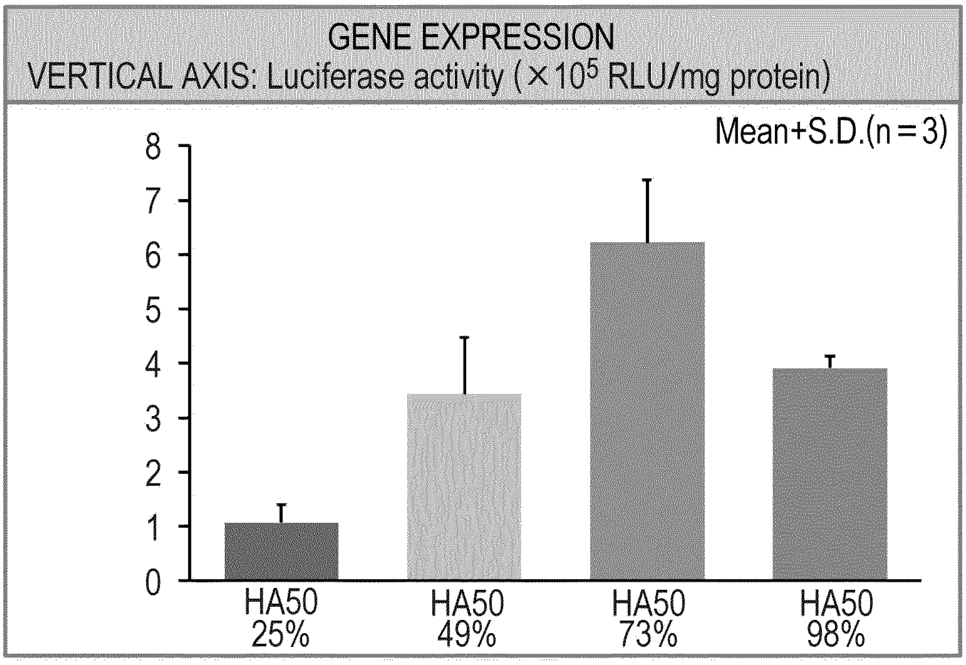
GENE EXPRESSION
VERTICAL AXIS: Luciferase activity (×10^5 RLU/mg protein)
Mean+S.D.(n=3)
8
7
6
5
4
3
2
1
0
HA50 25%
HA50 49%
HA50 73%
HA50 98%

FIG. 13

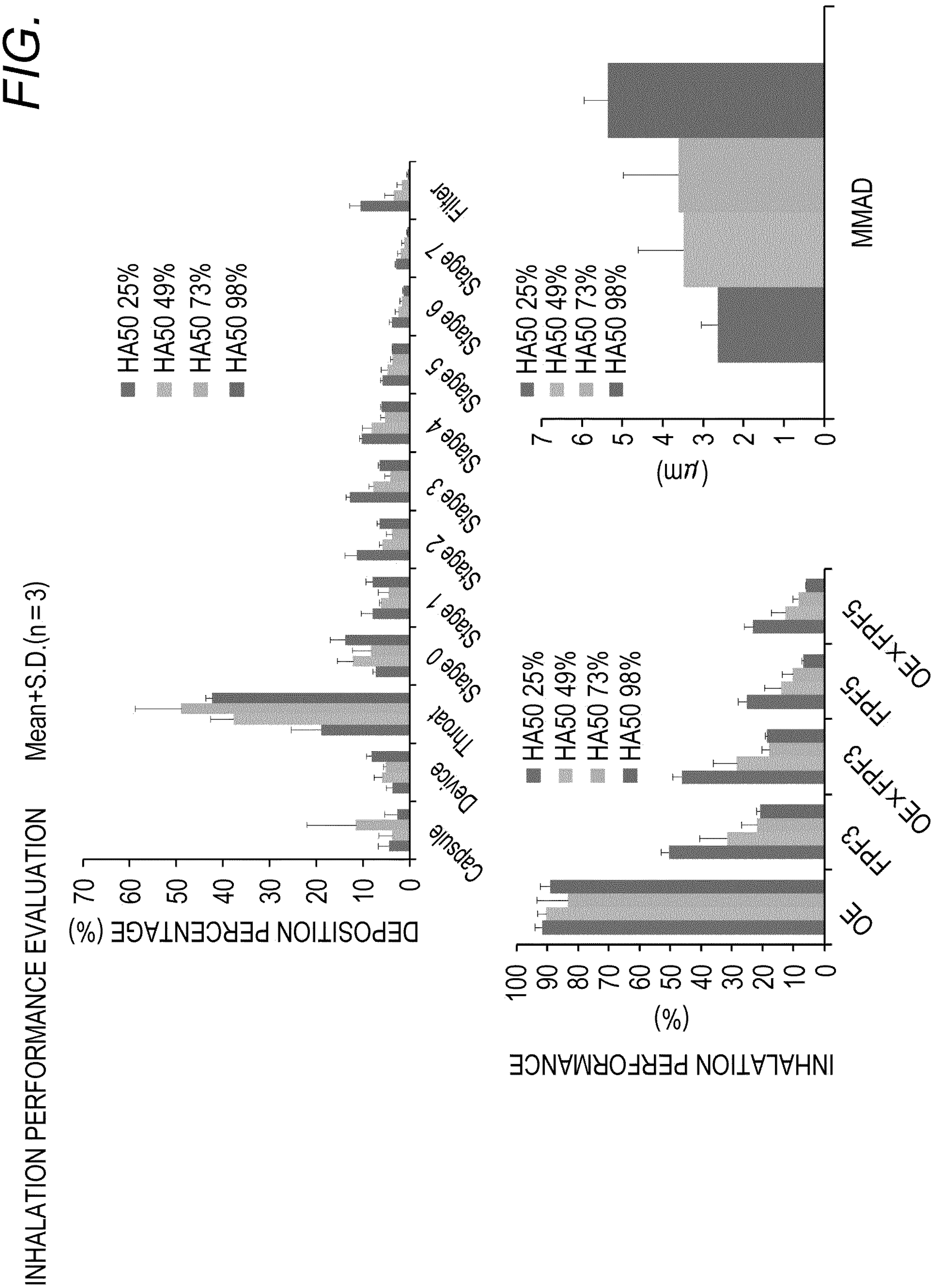
INHALATION PERFORMANCE EVALUATION
Mean+S.D.(n=3)
DEPOSITION PERCENTAGE (%)
0
10
20
30
40
50
60
70
Capsule
Device
Throat
Stage 0
Stage 1
Stage 2
Stage 3
Stage 4
Stage 5
Stage 6
Stage 7
Filter
HA50 25%
HA50 49%
HA50 73%
HA50 98%
INHALATION PERFORMANCE (%)
0
10
20
30
40
50
60
70
80
90
100
OE
FPF3
OE×FPF3
FPF5
OE×FPF5
HA50 25%
HA50 49%
HA50 73%
HA50 98%
(μm)
0
1
2
3
4
5
6
7
MMAD
HA50 25%
HA50 49%
HA50 73%
HA50 98%

FIG. 14

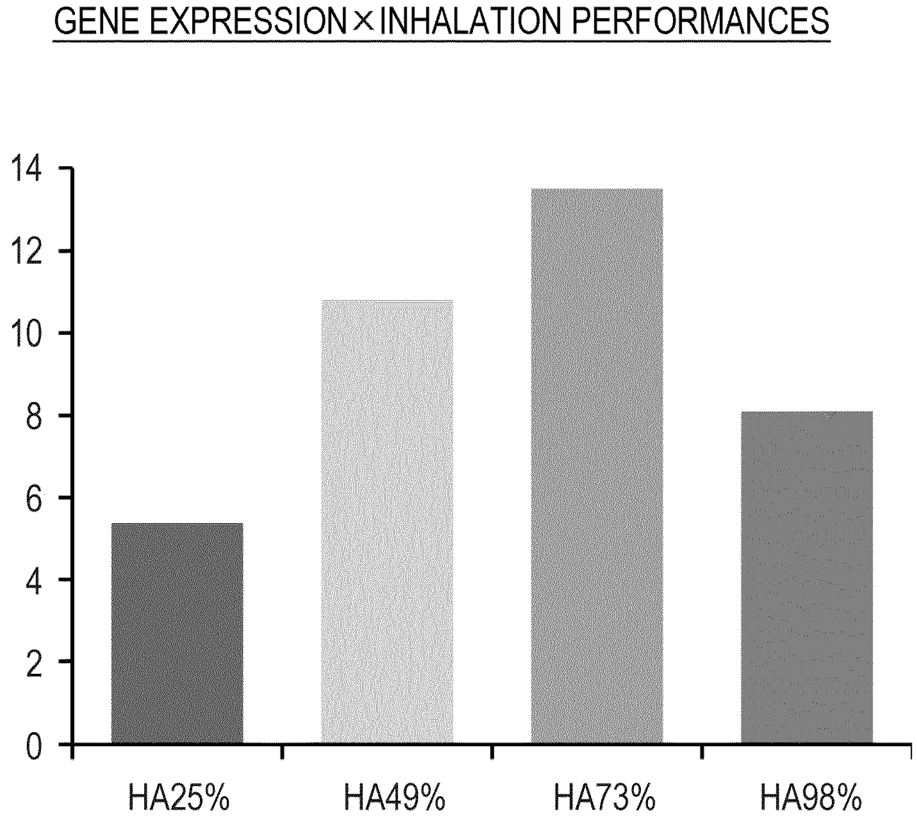
GENE EXPRESSION×INHALATION PERFORMANCES
14
12
10
8
6
4
2
0
HA25%
HA49%
HA73%
HA98%

**INTERNATIONAL SEARCH REPORT**

International application No. PCT/JP2019/038991

A. CLASSIFICATION OF SUBJECT MATTER
Int.Cl. A61K9/14(2006.01)i, A61K9/72(2006.01)i, A61K47/18(2006.01)i, A61K47/26(2006.01)i

According to International Patent Classification (IPC) or to both national classification and IPC

B. FIELDS SEARCHED

Minimum documentation searched (classification system followed by classification symbols)
Int.Cl. A61K9/14, A61K9/72, A61K47/18, A61K47/26

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| X | JP 2018-11588 A (MEIJO UNIVERSITY) 25 January 2018, claim 6, examples 1, 4, 6 (Family: none) | 1-24 |
| X | JP 2018-8942 A (PEARL THERAPEUTICS INC.) 18 January 2018, example 18 & US 2011/0135737 A1, example 18 & WO 2010/138862 A2 & EP 2435023 A2 & KR 10-2012-0015334 A & CN 102458364 A | 25-26 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 11.11.2019 | 19.11.2019 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2019/038991

C (Continuation). DOCUMENTS CONSIDERED TO BE RELEVANT

| Category* | Citation of document, with indication, where appropriate, of the relevant passages | Relevant to claim No. |
|---|---|---|
| X | JP 2002-531240 A (AVENTIS PHARMA LIMITED) 24 September 2002, paragraph [0016] & US 7041318 B2, column 3, second paragraph & WO 2000/032313 A1 & EP 1146964 A1 & CN 1328489 A | 27 |

Form PCT/ISA/210 (continuation of second sheet) (January 2015)

**INTERNATIONAL SEARCH REPORT**

International application No. PCT/JP2019/038991

**Box No. II Observations where certain claims were found unsearchable (Continuation of item 2 of first sheet)**

This international search report has not been established in respect of certain claims under Article 17 (2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
[see extra sheet]

1. ☐ As all required additional search fees were timely paid by the applicant, this international search report covers all searchable claims.
2. ☒ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

INTERNATIONAL SEARCH REPORT

International application No.
PCT/JP2019/038991

(Continuation of Box No. III)

(Invention 1) Claims 1-24

In document 1, there is described an inhalation agent or the like corresponding to "an inhalation powder that can be dispersed and pulverized by air absorption, and, can be swollen during moisture absorption, and contains an effective ingredient in at least a part of porous hollow spherical particles", and claims 1-24 lack in the novelty due to document 1 and does not have a special technical feature. Accordingly, claims 1-24 are classified as invention 1.

(Invention 2) Claims 25-26

Claims 25-26 cannot be said to have the technical feature same as or corresponding to claims 1-24 classified as invention 1.

Furthermore, claims 25-26 are not in the relationship substantially identical to or similarly closely related to any of claims classified as invention 1.

Therefore, claims 25-26 cannot be classified as invention 1.

And, since claims 25-26 have a special technical feature of "an evaluation method for an inhalation powder for acquiring a first aerodynamic mass median diameter calculated in the aspiration characteristics evaluation due to ACI and a second aerodynamic mass median diameter smaller than the first aerodynamic mass median diameter", claims 25-26 are classified as invention 2.

(Invention 3) Claim 27

Claim 27 cannot be said to have the technical feature same as or corresponding to claims 1-24 classified as invention 1 or to claims 25-26 classified as invention 2.

Furthermore, claim 27 is not in the relation substantially identical to or similarly closely related to any of claims classified as invention 1 or invention 2.

Therefore, claim 27 cannot be classified as any of invention 1 or invention 2.

And, since claim 27 has a special technical feature of "a method for measuring a mass change rate at least at 70%RH and a mass change rate at 95%RH when changed from 50%RH to 95%RH at 37°C in a dynamic moisture absorption measurement", claim 27 is classified as invention 3.

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2018187498 A
- JP 2017091941 A
- JP 2007522246 W
- JP 2018011588 A
- JP 2009155486 A


### Non-patent literature cited in the description


- Aerodynamic Particle Size Measurement for Inhalations. Supplement I to the Japanese Pharmacopoeia
- *Chem. Pharm. Bull.,* 2016, vol. 64, 239-245
- *National Institute of Pharmaceutical and Food Sanitation Report,* 2003, vol. 121, 30-33